(19) Europäisches Patentamt
European Patent Office
Office européen des brevets

(11) Publication number: **0 058 317**
**B1**

(12) **EUROPEAN PATENT SPECIFICATION**

(45) Date of publication of patent specification: **27.08.86**

(21) Application number: **82100585.7**

(22) Date of filing: **28.01.82**

(51) Int. Cl.[4]: **C 07 D 499/00,**
C 07 D 205/08,
C 07 D 471/04,
C 07 D 487/04,
C 07 D 498/04, C 07 F 7/18,
A 61 K 31/40, A 61 K 31/42,
A 61 K 31/43, A 61 K 31/545
// (C07D471/04, 221:00,
205:00),(C07D487/04, 209:00,
205:00),(C07D487/04, 235:00,
205:00),(C07D498/04, 263:00,
205:00)

(54) **Process for the preparation of 2-penem compounds.**

(30) Priority: **02.02.81 US 230774**

(43) Date of publication of application:
**25.08.82 Bulletin 82/34**

(45) Publication of the grant of the patent:
**27.08.86 Bulletin 86/35**

(84) Designated Contracting States:
**AT BE CH DE FR GB IT LI NL SE**

(56) References cited:
**EP-A-0 003 960**
**EP-A-0 013 662**
**EP-A-0 013 663**
**EP-A-0 018 305**
**EP-A-0 035 188**

(73) Proprietor: **SCHERING CORPORATION**
**2000 Galloping Hill Road**
**Kenilworth, New Jersey 07033 (US)**

(72) Inventor: **Afonso, Adriano**
**10 Woodmere Road**
**West Caldwell New Jersey 07006 (US)**
Inventor: **Hon, Frank**
**596 Paramus Road**
**Paramus New Jersey 07652 (US)**

(74) Representative: **Ritter, Stephen David et al**
**Mathys & Squire 10 Fleet Street**
**London EC4Y 1AY (GB)**

**EP 0 058 317 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

With reference to Belgian Patent Application No. 889,151, West German Patent Application No. 3122523, French Patent Application No. 2483924, British Patent Application No. 2078220 and Netherlands Patent Application No. 8102736 the Applicant has voluntarily submitted separate claims (of limited scope) for the foregoing countries.

The invention relates to a process for the preparation of compounds having the general formula (I)

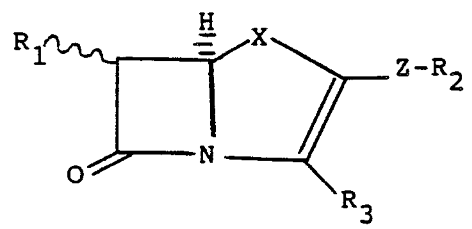

I

in which

$R_1$ is hydrogen, $C_1$—$C_6$ alkyl, acylamino or

$OR_5$
|
$R_4$—CH—,

wherein $R_4$ is hydrogen, $C_1$—$C_6$ alkyl, aryl, or phenyl substituted by $C_1$—$C_6$ alkyl, by $C_1$—$C_6$ alkoxy or by halogen, or heteroaryl said heteroaryl being an aryl group having a heteroatom in the ring and optionally having 1 to 3 $C_1$—$C_6$ alkyl substituents and $R_5$ is hydrogen or an hydroxy protecting group;

$R_2$ is $C_1$—$C_6$ alkyl, aryl, phenyl substituted by $C_1$—$C_6$ alkyl, by $C_1$—$C_6$ alkoxy or by halogen, $C_1$—$C_6$ alkyl substituted by one or more aryl groups, aminoalkyl, *N*-protected aminoalkyl, hydroxyalkyl, *O*-protected hydroxyalkyl, an optionally esterified, optionally *N*-protected α-amino acid residue or an optionally esterified carboxyalkyl group;

$R_3$ is nitrile, tetrazolyl or —$COOR_6$, wherein $R_6$ is hydrogen, —Alk—$C(Hal)_3$ (in which Hal is halogen and Alk represents a $C_1$—$C_6$ alkylene radical) $C_1$—$C_6$ alkyl, aryl, phenyl substituted by $C_1$—$C_6$ alkyl, by $C_1$—$C_6$ alkoxy or by halogen, an allylic group, an ester group which can be metabolically removed *in vivo*, or a carboxy protecting group;

X and Z are independently sulfur, oxygen, —$CH_2$—, —$CH_2$—$CH_2$— or the radical $=NR_7$ in which $R_7$ is hydrogen, carboxylic acid, acyl, $C_1$—$C_6$ alkyl, cycloalkyl containing 3 to 6 carbon atoms, aryl, phenyl substituted by $C_1$—$C_6$ alkyl, by $C_1$—$C_6$ alkoxy or by halogen, or an *N*-protecting group; and the pharmaceutically acceptable salts thereof.

For convenience the compounds of formula (I) wherein X is —$CH_2$— maybe identified as carbapenems; those in which X is —$CH_2$—$CH_2$— as carbacephams, and those in which X is sulfur, oxygen and $=NR_7$ respectively as penems, oxapenems and azapenems.

Certain novel compounds obtainable by the process of the invention are claimed in a divisional application, No. 851014209 (publication No. 0162193).

Some compounds of formula (I) in which X is sulfur and Z is S or —$CH_2$—, and from which any *O*- and *N*-protecting groups have been removed, have been proposed as antibacterial agents, and are disclosed, together with processes for their application, in for example, European Published Patent Application Nos: 13662 and 3960. Similar compounds were also described in EP—A 18305, EP—A 828, EP—A 5889 and (published in the priority interval of the present application) EP—A 35188.

The process described in European Published Application No. 3960, involves reacting a compound of the formula

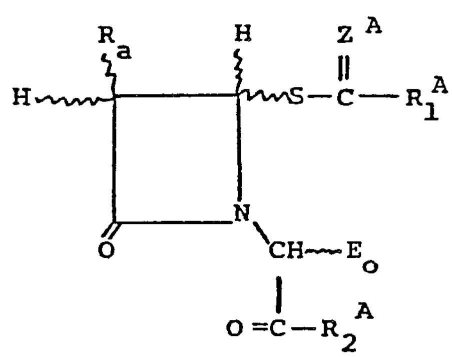

A

in which

$R_a$ is for example, an organic radical bonded by a carbon atom to the ring carbon atom, a free etherified or esterified hydroxy or mercapto group;

$Z^A$ is sulfur or oxygen;

$R_1{}^A$ is for example, an organic radical bonded by a carbon atom to the ring carbon atom or an etherified mercapto group, and $R_2{}^A$ forms with the carboxyl grouping —(C=O)— a protected carboxyl gorup, e.g., allyloxy and $E_0$ is a reactive esterified hydroxy group, e.g. chlorine, with a phosphine compound such as tri-lower alkyl phosphine or a triarylphosphine, or with a phosphite such as a tri-lower alkylphosphite; to form a phosphorarylidene of formula

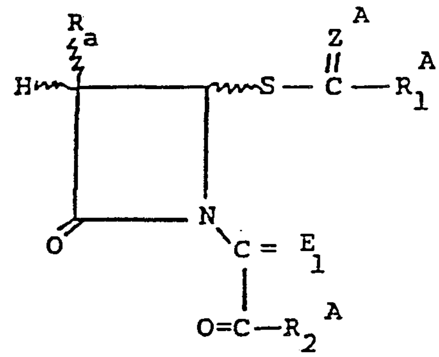

B

in which $E_1$ is a triaryl or tri-lower alkyl phosphoranylidene radical, or a phosphono compound of formula

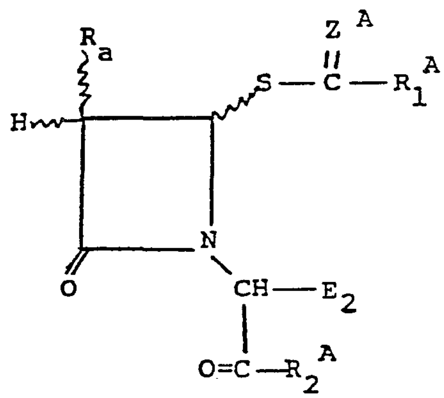

C

in which $E_2$ is e.g. dialkylphosphono. Ring closure occurs via the ylid compound of the formula

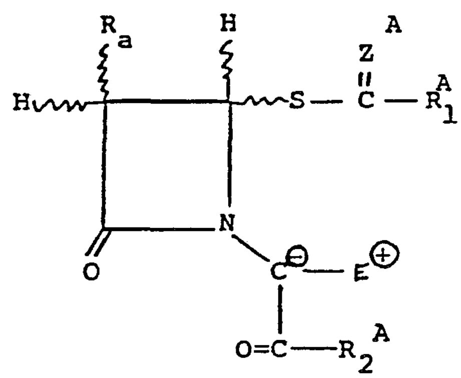

D

in which $E^{\oplus}$ represents a phosphono group three times or a phosphono group esterified twice with a cation.

Various routes are described for the preparation of the compounds of formula *A*. One such route involves reacting a compound of formula

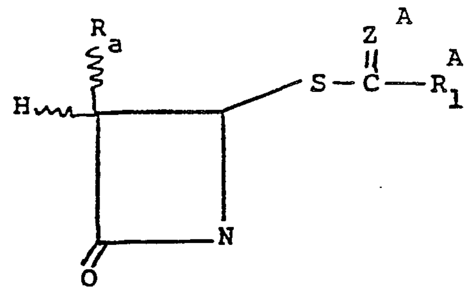

E

with a glyoxallic compound OHC—C(=C)—$R_2^A$ or a suitable derivative.

The disclosure also proposes the preparation of the intermediate compounds E by solvolysing a compound of formula

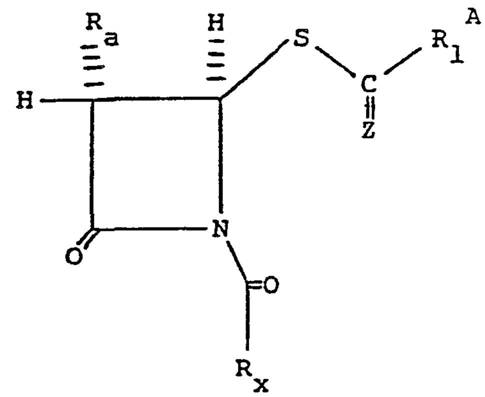

F

in which $R_x$ is e.g. an allyloxy carbonyl group.

Thus at least five stages are required in obtaining the desired compounds from intermediate F: each stage requiring the isolation and purification of the product before continuing on to the next stage, the overall operation requires well over a week to complete, and with a final product which is a mixture of compounds requiring several chromatographic purifications before the desired compound is isolated in pure form.

Similar procedures, requiring numerous steps, are described in the aforementioned European Published Patent Application No. 13,662 and in Journal of the American Chemical Society 100: 26,8214 (1978).

The process of the present invention comprises the preparation of a compound of formula (I) by reacting a compound of the general formula (II)

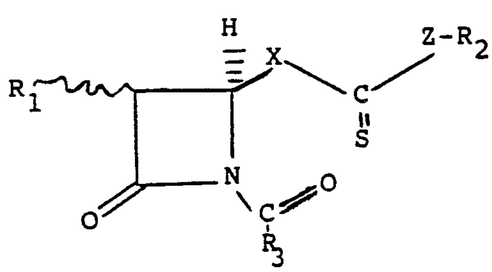

II

in which $R_1$, $R_2$, $R_3$, X and Z are as defined above wherein $R_5$ is a hydroxy protecting group, $R_7$ is not hydrogen, any carboxy group in substituent $R_2$ is esterified, and $R_6$ is not hydrogen; with an organo-substituted derivative of phosphorous acid and if required or desired, subjecting the resulting compound,

prior to or subsequent to isolation and any separation into its stereochemical isomers, if a mixture of isomers of compounds of formula II was subjected to the foregoing reaction, to one or more of the following operations:

a) removal of one or more protecting group;
b) conversion of an appropriate function into a free acid;
c) conversion of an appropriate function into a pharmaceutically acceptable salt; and
d) conversion of an appropriate function into a metabolisable ester group.

Some suitable organo substituted derivatives of phosphorous acid for use in the process of the invention are cyclic and/or acyclic trialkylphosphites, triarylphosphites and mixed alkyl arylphosphites or phosphoramides. The preferred organophosphorus compound is a trialkylphosphite and most preferably is triethyl phosphite.

Examples of suitable aryl and mixed arylalkyl phosphites are triphenyl phosphite; catechol phosphites, e.g. of the formula

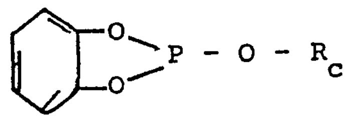

where $R_c$ is alkyl, e.g. methyl, ethyl, or aryl e.g. phenyl; and catechol dimer phosphites e.g.

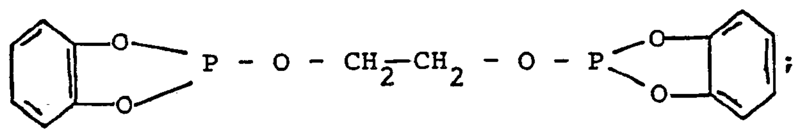

A suitable cyclictrialkyl phosphite is e.g.

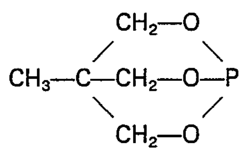

A suitable phosphoramide is one having the general formula

$$P(OR_D)_2\ N(R_E)_2$$

in which $R_D$ and $R_E$ are independently chosen from alkyl e.g. methyl, ethyl, and aryl e.g. phenyl.

Preferably the compound of formula (II) is one which has been prepared by the reaction of a compound of the following general formula III

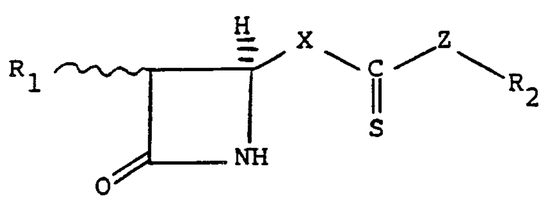

III

in which $R_1$, $R_2$, X and Z are as defined above with respect to formula II, with a reactive derivative of an acid of the following general formula (IV)

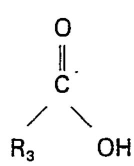

IV

preferably a halide thereof, in which the halogen is e.g. Cl or Br and $R_3$ is as defined above, the reaction being carried out in an inert solvent in the presence of an organic base, preferably a tertiary amine.

In the preceding reaction hydrogen halide is formed by the reaction and by decomposition of the acid halide.

In order to avoid decomposition of acid sensitive intermediates and final products (as for instance may occur when the conversion of a compound of formula III to a desired compound of formula I is carried out, without isolation of intermediates, in one vessel) it is advisable to add at a convenient stage in the preparation of the compound of formula I, an acid binding agent e.g. an alkaline earth metal bicarbonate or preferably an alkaline earth metal carbonate. Preferably the acid binding agent is included in the reaction mixture of the compound III and an acid halide.

The $C_1$—$C_6$ alkyl groups referred to above for $R_1$, $R_2$, $R_4$, $R_6$, $R_7$ and below for $R_{12}$, contain 1 to 6 carbon atoms and are exemplified by methyl, ethyl, propyl, butyl, pentyl, hexyl, and the corresponding branched-chain isomers thereof.

The acyl portion of the acylamino substituents referred to above for $R_1$ denotes acyl groups of the formula

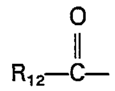

wherein the group $R_{12}$ is lower alkyl, aralkyl, lower alkoxy, aryloxy, alkenyl or alkynyl of 2—6 carbon atoms, cycloalkyl of 4—6 carbon atoms, heteroaryl or heteroaralkyl, optionally substituted by hydroxy, thiol, alkylthio, lower alkyl, lower alkoxy, halogen, cyano, carboxy, nitro, amino, mono- or di-alkylamino, mono- or di-acylamino, aminoloweralkyl and/or haloloweralkyl such as trifluoromethyl. Representative of such groups are those such as benzyl, p-hydroxybenzyl, 4-amino-4-carboxybutyl, methyl, cyanomethyl, 2-pentenyl, n-amyl, n-heptyl, ethyl, 3- or 4-nitrobenzyl, phenethyl, α, β, -diphenylethyl, methyldiphenylmethyl, triphenylmethyl, 2-methoxyphenyl, 2,6-dimethoxyphenyl, 2,4,6-trimethoxyphenyl, 3,5-dimethyl-4-isoxazolyl, 3-butyl-5-methyl-4-isoxazolyl, 5-methyl-3-phenyl-4-isoxazolyl, 3-(2-chlorophenyl)-5-methyl-4-isoxazolyl, 3-(2,6-dichlorophenyl), 5-methyl-4-isoxazolyl, D-4-amino-4-carboxybutyl, D-4-N-benzoylamino-4-carboxy-n-butyl, p-aminobenzyl, o-aminobenzyl, m-aminobenzyl, p-dimethylaminobenzyl, (3-pyridyl)methyl, 2-ethoxy-1-naphthyl, 3-carboxy-2-quinoxalinyl, 3-(2,6-dichlorophenyl)-5-(2-furyl)-4-isoxazolyl, 3-phenyl-4-isoxazolyl, p-carboxymethylbenzyl, m-fluorobenzyl, m-bromobenzyl, p-chlorobenzyl, p-methoxybenzyl, 1-naphthylmethyl, 3-isothiazolylmethyl, 4-isothiazolylmethyl, 5-isothiazolylmethyl, 4-pyridylmethyl, 5-isoxazolylmethyl, 4-methoxy-5-isoxazolylmethyl, 4-methyl-5-isoxazolylmethyl, 2-imidazolylmethyl, 2-benzofuranylmethyl, 2-indolylmethyl, 2-phenylvinyl, 2-phenylethynyl, 1-aminocyclohexyl, 2- and 3-thienylaminomethyl, 2-(5-nitrofuranyl) vinyl, phenyl, o-methoxyphenyl, o-chlorophenyl, o-phenylphenyl, p-aminomethylbenzyl, 1-(5-cyanotriazolyl)methyl, difluoromethyl, dichloromethyl, dibromomethyl, 1-(3-methylimidazolyl)methyl, 2- or 3-(4-carboxymethylthienyl)methyl, 2- or 3-(5-methylthienyl)methyl, 2- or 3-(methoxythienyl)methyl, 2- or 3-(4-chlorothienyl)methyl, 2- or 3-(5-carboxythienyl)methyl, 3-(1,2,5-thiadiazolyl)methyl, 3-(4-methoxy-1,2,5-thiadiazolyl)methyl, 2-furylmethyl, 2-(5-nitrofuryl)methyl, 3-furylmethyl, 2-thienylmethyl, 3-thienylmethyl, tetrazolylmethyl, and other acyl groups found in conventional penicillin derivatives, for instance cyclohexylamidinomethyl. The term also denotes an acyl residue derived from an α-amino acid of that L or D configuration.

"Halogen" refers to a fluorine, chlorine, bromine or iodine substituent. $R_8$ as halogen is preferably fluorine.

The term "heteroaryl" as used herein for $R_4$ refers to aryl groups having a hetero atom in the ring for example, pyridyl, furanyl and thienyl. The heteroaryl group may optionally contain 1 to 3 lower alkyl substituents, e.g., 2-methylpyridyl, 3-methylthienyl. Where there is a possibility of the various position isomers, the term "heteroaryl" is intended to cover all isomers, e.g., 2-pyridyl, 3-pyridyl and 4-pyridyl.

The term "metabolisable ester" group denotes an ester group which is metabolically removed in the body. Two particularly useful metabolisable ester gorups are the phthalidyl group and the pivaloyloxymethyl group.

Amine protecting groups, designated herein as *N*-protecting groups, and hydroxyl protecting groups, designated herein as *O*-protecting groups, and S-protecting groups, as well as their methods of preparation and removal are well known in the art.

Preferred *N*-protecting groups for use in the process of this invention to protect amine substituents included within the definitions of $R_2$, X and Y in formula I are groups such as 2,2,2-trichloroethoxycarbonyl, benzhydryloxycarbonyl or, preferably, allyl oxycarbonyl or 2-chloroallyl.

Preferred *O* and *S* protecting groups for use in the process of this invention are groups such as, 2,2,2-trichloroethoxycarbonyl, 1,1,1-trichloro-2-methyl-2-propoxycarbonyl, *p*-nitrobenzyloxycarbonyl, allyloxycarbonyl or 2-chloroallyl, with 2,2-trichloroethoxycarbonyl being most preferred. Suitable carboxy protecting groups are e.g. aralkyl groups conventionally used for this purpose e.g. benzyl or *p*-nitrobenzyl, and allylic groups, which can be readily removed by methods known in the art e.g. by hydrogenation in the case of aralkyl groups and e.g. by the McCombie method described below in the case of allylic groups.

Suitable 'allylic' groups for $R_6$ (in which they perform a carboxy protective function) are any of those having the allylic structure, e.g. allyl, haloallyl, methyl allyl and crotyl. The preferred allylic group is chloroallyl, most preferably 2-chloroallyl.

The reaction of the compound of formula (II), and also the reaction of the azetidinone of formula (III) with the acid halide, are conveniently carried out in inert solvent. By "inert solvent" is meant any organic or inorganic solvent in which the starting compound and reagents are soluble and which will not interfere with the process under the reaction conditions thereof, so there are produced a minimum of competing side reactions. Inert solvents which may be used in our process include aromatic hydrocarbons (e.g. benzene, toluene and the like), aliphatic ethers (e.g. diethyl ether, dipropyl ether), cyclic ethers (e.g. dioxane, tetrahydrofuran) and, preferably halogenated hydrocarbons such as methylene chloride and chloroform.

In general those inert solvents are preferred (e.g. halogenated hydrocarbons) which have the solubility characteristics discussed hereinabove and which have a boiling point in the range of 40°C to 80°C so that when the compounds of formula II which are used are obtained by the process defined herein, the same solvent may be used both in the preparation of the compound II, and the reaction thereof with a trivalent organophosphorus compound, and the entire operation may be advantageously carried out in one vessel without the necessity of isolating the compound II.

In the practice of the process of the invention, usually the imido compound II is reacted in an inert solvent with two molar equivalents of a trialkylphosphite (usually triethylphosphite) solvent and conveniently a solution of the trialkylphosphite in inert solvent is added to a solution of the compound II in an inert solvent; the reaction solution is maintained at temperatures in the range of from 20°C to 80°C, as specified above, usually from 40°C to 60°C, usually for a period of from 6 to 24 hours.

Greater yields of *N*- and *O*-protected compounds of formula I are obtained when a trialkylphosphite solution is added to a solution of compound II over a period of from 2 to 3 hours. Best yields of the product of formula I are also obtained when the reaction is carried out at 40°C to 60°C. When T.L.C. shows the absence of imido intermediate, II the desired product (i.e. compound of formula (I)) can be isolated and purified via conventional techniques, usually chromatographic techniques followed by crystallization.

In general, the preparation of a compound of formula (II) preferably comprises the reaction of an azetidinone of formula (III), in an inert solvent (as defined hereinabove), usually at a temperature in the range of from 5°C to 25°C, preferably from 10°C to 15°C, usually with an equimolar amount of an acid halide derivative of the acid of formula IV and a tertiary amine, in the presence of an alkaline earth metal carbonate, preferably calcium carbonate, in an amount which is at least equimolar (and preferably is in excess of equivalent) to the azetidinone of formula (III).

Any tertiary amine (e.g. triethylamine) may be used in our process with di-isopropyl-ethylamine being preferred.

In carrying out the N-acylation of the azetidinone of formula (III) usually about 1.2 moles of each of the tertiary amine and a derivative of the acid of formula (IV) and a large excess of calcium carbonate (e.g. 10 moles) are used per mole of the azetidinone (III) with a molar ratio of solvent to azetidinone (III) of 10:1.

In a preferred embodiment an acid halide is used which is allyl oxalylchloride or preferably chloroallyloxy, oxalylchloride, the tertiary amine is di-isopropylethylamine, and the alkaline earth metal carbonate is calcium carbonate.

The N-acylation of the azetidinone (II) can be monitored by thin layer chromatography (TLC) until no starting material is detected. At that point, the reaction solution containing imido intermediate (II) may be filtered to remove any acid binding agent and the filtered solution washed with water to remove the tertiary amine salt formed during the reaction, then dried and diluted with additional inert solvent prior to reaction with a trialkylphosphite. Alternatively, the reaction mixture may be diluted directly with additional inert solvent so that the molar ratio of starting azetidinone (III) (and thus, also, of the imido intermediate II) to solvent is about 1 to 50.

The compounds of formula (I) possess several centres of chirality and the process of this invention, depending on the configuration of the starting compound, will produce either chiral compounds of a specific configuration or isomeric mixtures.

The compounds of formula (I) may be prepared as their racemic mixtures, e.g., a 5R,6S,8R compound is produced with its enantiomer (mirror image), i.e., a 5S,6R,8S compound, in equal amounts when the starting compound of formula II is a racemic mixture. The two enantiomers may be separated by conventional means, e.g., by fractional crystallizations of optically active salt forms, e.g., the salts derived from optically active amino compounds, e.g., (−)-brucine, or (+)- and (−)-ephedrine.

Alternatively, the compounds may be produced in their pure enantiomeric forms by utilizing optically active starting materials of formula II in the synthesis procedure.

A preferred aspect of this invention is directed to a process for preparing compounds of formula (I) wherein $R_1$ is

$$\begin{array}{c} OR_5 \\ | \\ R_4\text{—}CH\text{—}, \end{array}$$

$R_2$ is methyl or ethyl and Z and X are both sulfur, particularly the preparation of the foregoing compounds wherein $R_4$ is methyl and $R_5$ is a hydroxy protecting group or preferably hydrogen. The preferred

configuration of the foregoing compounds is that wherein the configuration at C-5 and C-6 is of the absolute stereochemistry R and S, respectively. The two hydrogen atoms attached to the 5 and 6 carbon atoms are thus *trans* to one another. The stereochemistry of the C-8 carbon atom (i.e. the carbon atom of

$$\begin{array}{c} OR_5 \\ | \\ R_4\text{—}CH\text{—group)} \end{array}$$

may be designated as either R or S depending on the exact nature of the $R_2$ substituent. For instance, the compounds wherein $R_4$ is methyl will have the 8R stereochemistry. The most preferred embodiment of the process aspect of this invention is, thus concerned with the preparation of compounds of the following formula having a stereoconfiguration designated 5R,6S,8R and having the following representative spatial configuration

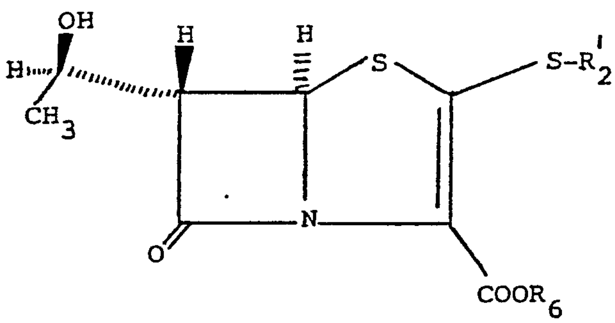

wherein $R_2^1$ is methyl or ethyl.

A preferred embodiment of our process, will be described with reference to the following flow diagram;

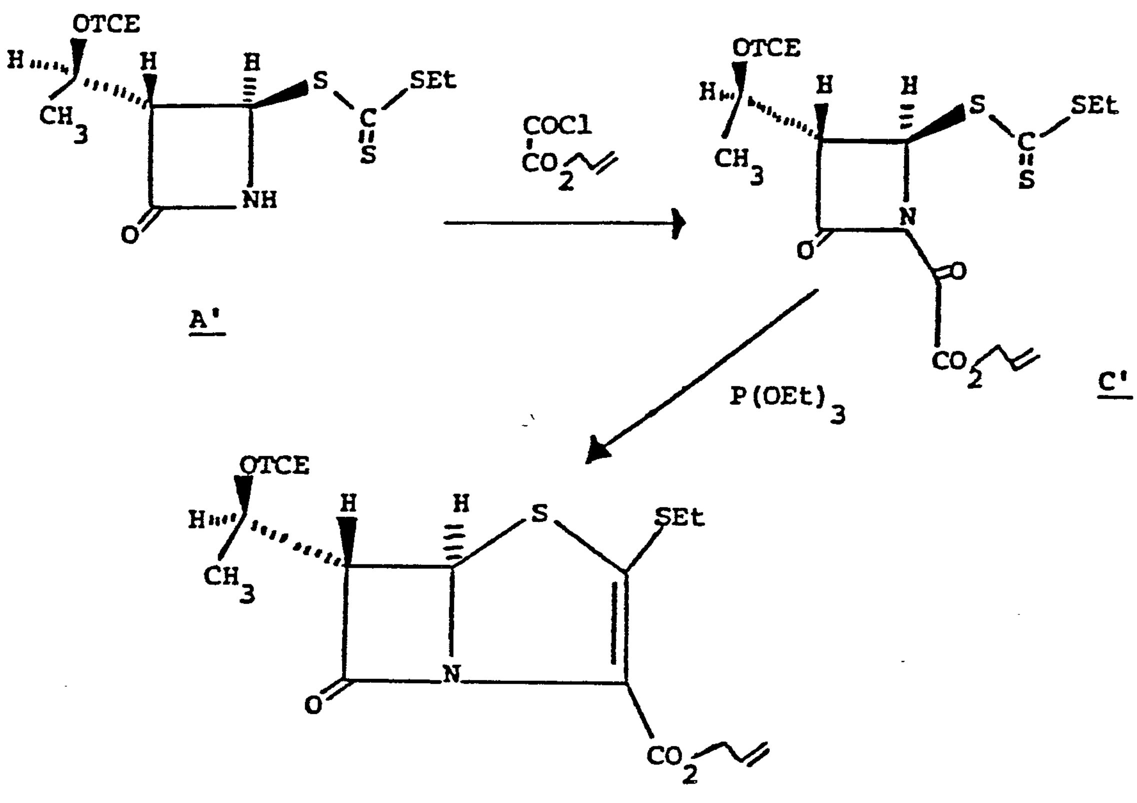


A'

C'

I'

In carrying out this preferred operation in accordance with the present invention, to the azetidinone of formula A' (which is a compound of formula II where $R_1$ is

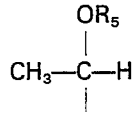

wherein $R_5$ is the *O*-protecting group trichlorethoxycarbonyl, represented by "TCE" in formula A'), having the stereoconfiguration 3S,4R,5R, in a chloroform solution at 10°C. to which has been added molar excess of calcium carbonate with respect to the azetidinone A', there is added 1.2 molar equivalents, with respect to the azetidinone A', of allyloxy oxalylchloride (i.e. a compound of formula (III) wherein $R_3$ is carboxyallyl) followed by 1.2 molar equivalents, with respect to the azetidinone A', of di-isopropylethylamine in methylene chloride. After 15 minutes reaction time, the excess calcium carbonate is filtered off, the organic solution is washed with water, and chloroform is added to made a 50:1 ratio of chloroform to starting compound. The chloroform solution of the imido intermediate C' is then brought to reflux temperature at which temperature there is added thereto, over a 3 hour period, a solution of 2 equivalents of triethylphosphite in chloroform. After refluxing an additional 18 hours, the desired compound of formula I', i.e. allyl-(5R,6S,8R)-6-[1-(2,2,2-trichloroethoxycarbonyloxy-ethyl)]-2-(ethylthio)penem-3-carboxylate is isolated via chromatography on silica gel and crystallization from ether-hexane.

The foregoing product can then be treated to obtain a bacterially active compound by removing the *O*-protecting group at C-8 (i.e. the trichloroethoxycarbonyl) by known procedures e.g. via zinc/acetic acid; and removing the allyl protecting group of the 3-carboxylic acid function. The allyl group is most preferably removed by the procedure of McCombie, described in E.P.O. Published Application No. 0013663. The McCombie deprotection procedure utilizes a suitable aprotic solvent, such as tetrahydrofuran, diethyl ether or methylene chloride, with potassium or sodium 2-ethylhexanoate or 2-ethylhexanoic acid and a mixture of a palladium compound and triphenyl phosphine as the catalyst; the use of potassium or sodium 2-ethylhexanoate provides the corresponding salt, while use of 2-ethylhexanoic acid affords the free acid, i.e. the compound sodium or potassium, 5R,6S,8R-6-(1-hydroxyethyl)-2-ethylthio-2-penem-carboxylate or the free acid thereof.

The McCombie deprotection method is particularly suitable for sensitive betalactam carboxylates according to this invention.

By following the foregoing procedure and treating allyl-(5R,6S,8R)-6-[1-(2,2,2-trichloroethoxycarbonyloxy-ethyl)]-2-(methylthio)-penem-3-carboxylate, sodium 5R,6S,8R-6-(1-hydroxyethyl)-2-methylthio-2-penem-carboxylate or the corresponding free acid is prepared.

The aforesaid deprotection procedures can be used in preparing the salts and free acids of other compounds within the scope of the invention, e.g. of those compounds defined with respect to formulae (VI) or (VIII).

The compounds of formula (I) wherein $R_6$ is a metabolisable ester group can be prepared directly or by reaction of the corresponding alkali metal salt with the corresponding halide such as chlorophthalide or pivaloyloxymethyl chloride in a solvent such as dimethyl formamide. Preferably a catalytic amount of sodium iodide is added.

The process of our invention, as described above, represents an improvement over the previously mentioned prior art methods for the conversion of the imido compounds to compounds of formula (I). By our process the conversion of an imido compound of formula (II) to compound of formula I can be effected in a single operation: likewise the conversion of azetidinone compounds to the imido intermediate is effected in a single step. In preferred embodiments of our process the conversion of an azetidinone to the desired final product, in these two steps, can be carried out in a single reaction vessel and completed in less than one day. It will also be noted that our process does not involve an ylid intermediate such as of formula D in the prior art process discussed above.

The cyclisation of the process of this invention is believed to proceed via a carbene mechanism involving a transitory carbenoid or carbene derived from the imido compound II upon reaction with a trivalent organophosphorus compound, for instance in the preferred embodiment of our process described above the carbenoid and carbene are believed to have the following structures J' and K' respectively.

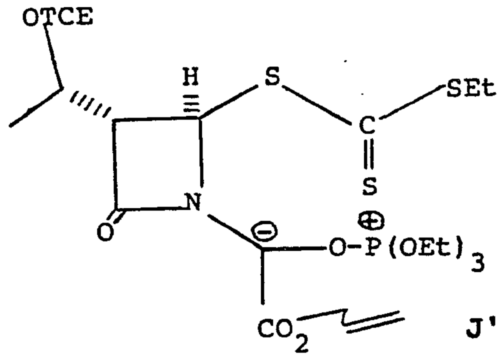

J'

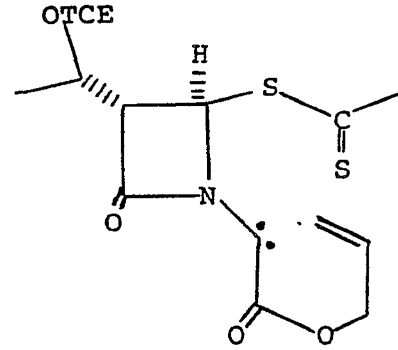

K'

Compounds II and III for our process are either known compounds or can be prepared by procedures known in the art. Thus, azetidinones of formula III wherein X sulfur, such as described in further detail in the preparations and Examples and the discussion of the preferred process species of this invention are described in E.P.O. Published Application Nos: 13,662 and 3,960. Other azetidinone and imido starting compounds are also known or can be prepared via known procedures.

Representative compounds which can be produced by using the process of this invention are as follows:

(5R,6S,8R) sodium-2-(isopropylthio)-6-(1-hydroxyethyl)-2-penem-3-carboxylate,

Disodium (5R,6S,8R)-2-(3-carboxy-1-propylthio)-6-(1-hydroxyethyl)-2-penem-3-carboxylate,

Potassium (5R,6S,8R)-2-(t-butylthio)-6-(1-hydroxyethyl)-2-penem-3-carboxylate,

Sodium (5R,6S,8R)-2-ethoxy-6-(1-hydroxyethyl)-2-penem-3-carboxylate,

Sodium (5R,6S,8R)-2-butoxy-6-(1-hydroxyethyl)-2-penem-3-carboxylate,

Sodium (5R,6S,8R)-2-propoxy-6-(1-hydroxyethyl)-2-penem-3-carboxylate,

(5R,6S,8R)-2-(2'-aminoethoxy)-6-(1-hydroxyethyl)-2-penem-3-carboxylic acid,

Sodium (5R,6S,8R)-2-methoxy-6-(1-hydroxyethyl)-2-penem-3-carboxylate,

(5R,6S,8R,2'S)-2-[(2'-amino-2'-carboxyethyl)thio]-6-(1-hydroxyethyl)-2-penem-3-carboxylic acid pyridinium or sodium salt,

Disodium (5R,6S,8R)-2-(2'-carboxyethylthio)-6-(1-hydroxyethyl)-2-penem-carboxylate,

Sodium (5R,6S,8R)-2-(2'-hydroxyethylthio)-6-(hydroxyethyl)-2-penem-3-carboxylate,

Sodium (5R,6S,8R)-2-(2'-ethylthio)-6-(1-fluoroethyl)-2-penem-3-carboxylate,

and the various compounds within the scope of Formula I as defined above which are disclosed in our European Patent Applications publication nos. 0013662 and 0035188.

Certain compounds of formula I possess antibacterial activity, including novel compounds defined by formula VI to VIII. The antibacterially active compounds are those of the aforesaid formulae but in which, when $R_1$ contains an *O, S* or *N* protecting group and/or $R_2$ contains an O-protecting group or an N-protecting group and/or $R_7$ is an N-protecting group then $R_3$ is chosen from nitrile, tetrazolyl, and —$COOR_6$ in which $R_6$ is H, the radical $C(Hal)_3$—Alk in which Hal is halogen and Alk represents an alkylene radical, or a metabolisable ester group; and the pharmaceutically acceptable salt thereof.

The aforementioned antibacterially active compounds of the invention are active against both gram-positive organisms such as *Staphylococcus epidermis* and *Bacillus subtilis*, and such gram-negative organisms as *E. coli* and *Salmonella.*

Thus the present invention includes within its scope pharmaceutical compositions comprising an antibacterially active compound or a pharmaceutically acceptable salt thereof as defined in the second preceding paragraph, together with a pharmaceutically acceptable carrier or excipient.

The dosage administered of the antibacterially active compounds of this invention is dependent upon the age and weight of the animal species being treated, the mode of administration, and the type and severity of bacterial infection being prevented or reduced. Typically, the dosage administered per day will be in the range of 5 to 200 mg/kg with 20 to 80 mg/kg being preferred.

For oral administration, the antibacterially active compounds may be formulated in the form of tablets, capsules, elixirs or the like. Likewise, they may be admixed with animal feed. They may also be applied topically in the form of ointments, both hydrophilic and hydrophobic, in the form of lotions which may be aqueous, non-aqueous or of the emulsion type, or in the form of creams.

Also they may be utilized in liquid form such as solutions, suspensions and the like for otic and optic use any may also be administered parenterally via intramuscular injection.

The Preparations and Examples which follow, illustrate the process and novel compounds of the present invention. Throughout these Preparations and Examples, "NMR" deontes nuclear magnetic resonance spectra; "rotation" denotes optical rotation of the compounds in a suitable solvent; "MS" denotes mass spectra; "UV" denotes ultraviolet spectra; and "IR" denotes infra-red spectra. Chromatography is performed on silica gel unless otherwise noted.

## Preparation A

Allyloxalyl chloride

Allyl alcohol (11.6 g) is added dropwise with stirring to a cold (0°C) solution of oxalyl chloride (25.4 g) in dry ether (50 ml) while maintaining the temperature of the reaction mixture during the addition at 10°C—12°C. The reaction mixture is then stirred overnight followed by removal of the solvent in a rotary evaporator. The resultant residue is distilled to yield allyloxalyl chloride as a colourless liquid (16 g), b.p. 68°C—70°C/44 mm.

## Preparation B

(3S,4R,5R)-3-[1-(2,2,2-trichloroethoxycarbonyloxy-ethyl)]-4-[(ethoxy)carbonothiolythio]-azetidin-2-one

Carbon disulfide (4 ml) is added dropwise, to a solution of ethanol (50 ml) containing 1-N sodium hydroxide (10 ml) and the mixture is stirred for ten minutes after which it is added dropwise to a solution of (3S,4R,5R)-[1-(2-methoxy-1,2-dioxoethyl)]-3-[1-(2,2,2-trichloroethoxycarbonyl-oxyethyl)]-4-chloroazetidin-2-one (4.1 g) in ethanol. The reaction mixture is stirred until t.l.c. analysis indicates no starting compound is present (about 4 hours) then diluted with ethyl acetate; the solution is washed with saturated sodium chloride, dried over magnesium sulfate and evaporated. The resulting residue is chromatographed on silica gel (40 g) eluting with 30% ether-hexane. The like elutes, as determined by the t.l.c., are combined and evaporated to a residue which is the title compound. I.R. = 5.65 μ;

NMR: δ 5.5 ppm (1H, d, J=2 cps)
3.4 ppm (1H, q, J=8 and 2 cps).

## Example 1

Allyl (5R,6S,8R)-6-[1-(2,2,2-trichloroethoxy-carbonyloxyethyl)]-2-ethylthio-2-penem-3-carboxylate

To a solution of (3S,4R,5R)-3-[1-(2,2,2-trichloroethoxyethylcarbonyloxyethyl)]-4-[(ethylthio)-carbonothioylthio]-azetidine-2-one (0.628 g) in methylene chloride (6 ml) cooled to 10°C, is added with stirring, calcium carbonate (0.6 g) followed by allyloxalyl chloride (0.263 g, 1.2 eq.). A solution of diisopropylethylamine (0.32 ml, 1.2 eq.) in methylene chloride (1 ml) is added thereto over a period of 5 mins., while maintaining the temperature in the range of 10°C—15°C. After TLC shows no starting compound (after say 15 mins. at 15°C), the mixture is transferred to a separatory funnel using ethanol-free chloroform. One solution is washed twice with ice/water, filtered to remove excess calcium carbonate, dried over anhydrous sodium sulfate, and transferred to a 100 ml 3-neck flask. The volume of the solution is adjusted to approximately 50 ml with chloroform, and heated at reflux temperature while adding a solution of triethylphosphite (0.6 ml, 2 eq.) in chloroform (20 ml) over a 3 hour period. The mixture is refluxed for an additional 18 hours, evaporated and chromatographed on 14 g silica gel, eluting with 25% ether-hexane. The like elutes are combined and evaporated to obtain a residue (420 mg) comprising the title compound (58% yield). The residue is purified by crystallization from ether-hexane to obtain the title compound in crystalline form. Yield 330 mg (46% theory).

## Example 2

Allyl (5R,6S,8R)-6-[1-(2,2,2-trichloroethoxycarbonyloxyethyl)]-2-ethoxy-2-penem-3-carboxylate

Allyloxalylchloride (0.52 g) is added, with stirring, to a cold solution of (3S,4R,5R)-3-[1-(2,2,2-trichloroethoxycarbonyloxyethyl)]-4-[(ethoxy)-carbonothioylthio]-azetidin-2-one in methylene chloride (10 ml) containing calcium carbonate (1.0 g). A solution of di-isopropyl-ethylamine (0.62 ml) in methylene chloride (2 ml) is added to this reaction solution at a rate so as to maintain the reaction temperature between 10°C—15°C. After 15 mins., the solution is washed 2 times with cold saturated sodium chloride, filtered, dried, transferred to a 3-neck flask and diluted to 45 ml with methylene chloride. The solution is refluxed while adding a solution of triethylphosphite (1.3 ml) in methylene chloride (15 ml) over a period of 3.6 hours. The reaction mixture is refluxed for an additional 17 hours, cooled and evaporated. The resulting residual oil is chromatographed on 30 g silica gel and eluted with 35% ether-hexane to obtain the title compound. The product is purified by crystallization from ether-hexane to obtain colourless needles (300 mg).

mp 84°C—85°C $[\alpha]_D$ + 167° (chloroform).
I.R.: 5.60, 5.75 μ.
NMR ($CDCl_3$) δ 5.55 ppm (d, 1H, J=1.5 cps)
4.22 ppm (q, 2H).
MS: $M^+$ 474.

## Preparation C

(3S,4R,5R)-3-[1-(2,2,2-trichloroethoxycarbonyloxy-ethyl)]-4-[(methoxy)-carbonothioylthio]-azetidin-2-one

By following the procedure of Preparation B using methanol in place of ethanol, there is obtained the title compound as colourless crystals. m.p. 97°C—99°C., I.R.: 5.65 μ.

NMR: δ 4.2 ppm (3H, S)
5.47 ppm (1H, d, J=2.5 cps)
3,4 ppm (1H, q, J=7; 2.5 cps).

Preparation D

(3S,4R,5R)-3-[1-(2,2,2-trichloroethoxycarbonyloxy-ethyl)]-4-[(propoxy)-carbonothioylthio]-azetidin-2-one

By following the procedure of Preparation B using propyl alcohol in place of ethanol, there is obtained the title compound as a colourless oil. I.R.: 5.65 μ.

NMR: δ 5.45 ppm (1H, d, J=2.0 cps)
3.4 ppm (1H, q, J=8; 2.0 cps)
4.57 ppm (2H, t, J=6 cps).

Preparation E

(3S,4R,5R)-3-[1-(2,2,2-trichloroethoxycarbonyloxy-ethyl)]-4-[(butoxy)-carbonothioylthio]-azetidin-2-one

By following the procedure of Preparation B using butyl alcohol in place of ethanol, there is obtained the title compound as a colourless oil. I.R.: 5.65 μ.

NMR: 5.47 ppm (1H, d, J=2.5 cps)
3.4 ppm (1H, q, J=8; 2.5 cps)
4.61 ppm (2H, t, J=7 cps).

Preparation F

(3S,4R,5R)-3-[1-(2,2,2-trichloroethoxycarbonyloxy-ethyl)]-4-[(2'-allyloxycarbonylamino)ethoxy-carbonothioylthio]-azetidin-2-one

By following the procedure of Preparation B using N-allyloxycarbonylaminoethanol in place of ethanol, there is obtained the title compound.

NMR: 5.44 ppm (1H, d, J=2.4 cps)
3.38 ppm (1H, q, J=6.6; 2.4 cps).
$[\alpha]_D$ + 114° (chloroform).

Preparation G

(3S,4R,5R,2'S)-3-[1-(2,2,2-trichloroethoxycarbonyloxy-ethyl)]-4-[(2'-allyloxycarbonylamino-2'-allyloxycarbonylethyl-thio)-carbonothioylthio]azetidin-2-one

A solution of bis-(N-allyloxycarbonyl)-D-cystine bis-allyl ester (14.76 g) in methanol (150 ml) containing zinc dust (15 g) is cooled to 0°C and stirred while adding concentrated hydrochloric acid (10.4 ml) at a rate to maintain the temperature 0—6°C. Following the addition of the acid, the mixture is stirred for an additional 2 minutes, poured on ice/water (200 ml), filtered and the organic layer of the filtrate is washed with water, dried over sodium sulfate and evaporated to dryness. The resulting oil is dissolved in ethanol (140 ml), treated with 1N sodium hydroxide (60.5 ml) followed by carbon disulfide (30 ml) and the resulting solution is added dropwise with stirring to a solution of (3S,4R,5R)-3-[1-(2,2,2-trichloroethoxycarbonyloxy-ethyl)]-4-acetoxyazetidinone (19 g) in ethanol (100 ml) at −15°C so that at the end of addition, the reaction mixture temperature is about 1°C. After about 45 minutes at this temperature and when thin layer chromatography indicates disappearance of starting material, the reaction is worked up by pouring in ice/water (200 ml), extraction with ethylacetate (400 ml), washing the extract with brine, drying and evaporating. The resulting viscous yellow liquid is chromatographed on silica gel (500 g). Elution with 20% ethylacetate/hexane affords the title compound as an orange yellow glass.

I.R.: 5.6, 5.7, 5.8 μ
$[\alpha]_D$ + 139° (chloroform).
NMR ($CDCl_3$) 1.5 (d, 3H, J=6 cps)
3.42 (dd, 1H, J=2; 7 cps)
5.65 (d, 1H, J=2 cps).

Preparation H

O-ethyl N-cyclohexyl N-[{4-(3S,4R,5R)-3-[1-(2,2,2-trichloroethoxycarbonyloxyethyl)]azetidin-2-one-yl}]thiocarbamate

A solution of dicyclohexylcarbodiimide (0.6 g) and sodium ethoxide (0.2 g) in tetrahydrofuran (20 ml) is stirred at room temperature for one hour. The solution is then cooled while adding a solution of (3S,4R,5R)-3-[1-(2,2,2-trichloroethoxycarbonyloxy-ethyl)]-4-acetoxyazetidin-2-one (1 g) in tetrahydrofuran. After stirring for 3 hours hydrogen sulfide gas is bubbled through the solution for one hour followed by evaporation to remove the solvent. The residual oil upon chromatography affords the title compound.

Preparation I

Ethyl N-cyclohexyl N-[4-{(3S,4R,5R)-3-[1-(2,2,2-trichloroethoxycarbonyloxyethyl)] azetidin-2-one-yl}]dithiocarbamate

A solution of dicyclohexylcarbodiimide (0.6 g) in tetrahydrofuran (6 ml) is heated with sodium ethylmercaptide (0.25 g). The solution is stirred for 30 minutes and then is treated with a solution of (3S,4R,5R) 3-(2,2,2-trichloroethoxycarbonyloxyethyl)-4-acetoxy azetidin-2-one (1 g) in tetrahydrofuran. The solution is stirred for 3 hours; dry hydrogen sulfide gas is then bubbled through the reaction mixture for 1 hour and the solvent is then removed under pressure. The residual oil upon chromatography on silica gel affords the title compound.

Preparation J

N,N'-diacetyl N'-[4-{(3S,4R,5R)-3-[1-(2,2,2-trichloroethoxycarbonyloxy-ethyl)] azetidin-2-on-yl}]thiourea

A suspension of sodium hydride (0.75 g) in tetrahydrofuran (10 ml) containing N,N'-diacetyl S-trityl isothiourea (1.2 g) is stirred under nitrogen for 2 hours after which (3S,4R,5R) 3-(-trichloroethoxycarbonyloxyethyl) 4-acetoxy azetidin-2-one (1.0 g) is added to it. Stirring is continued for an additional 1 hour and the reaction mixture is then diluted with 1.1N HCl/ice and extracted with ethylacetate. The extract is concentrated under reduced pressure and the title compound is isolated by chromatography on silica gel.

Preparation K

N-acetyl S-[4{(3S,4R,5R) 3-[1-(2,2,2-trichloroethoxycarbonyloxyethyl)] azetidin-2-on-yl}]dithiocarbamate

A solution of acetamide (0.2 g) in tetrahydrofuran (2 ml) is stirred under nitrogen with sodium hydride (0.07 g) at 55°C. for 1 hour. The solution is then cooled to room temperature, and carbondisulfide (0.5 ml) followed after 15 minutes by (3S,4R,5R) 3-[1-(2,2,2-trichloroethoxycarbonyloxyethyl)] 4-acetoxy azetidin 2-one (1.0 g) are added. The reaction mixture is stirred for 1 hour and then diluted with water/ice and extracted with ethyl acetate. The extract is concentrated under reduced pressure and the title compound is isolated by chromatography on silica gel.

Preparation L

(3S,4R,5R) 3-[1-(2,2,2-trichloroethoxycarbonyloxyethyl)] 4-[2-{(β-allyloxycarbonylaminoethylthio)-thiocarbonyl}-methyl] azetidin 2-one

A solution of (3S,4R,5R) 3-[1-(2,2,2-trichloroethoxycarbonyloxyethyl)]-4-carboxymethyl azetidin-2-one (1.0 g) in ethyl acetate (10 ml) containing 2-allyloxycarbonylamino ethanethiol (0.5 g) is cooled to 0°C. and heated with dicyclohexylcarbodiimide (0.6 g). The reaction mixture is allowed to stand at 0°C. for 24 hours and then filtered. The filtrate is evaporated to dryness and the residual oil is re-dissolved in toluene (20 ml) containing Lawesson's reagent* (1.2 g). The solution is heated for 3 hours followed by chromatography on silica gel to isolate the title compound.

* 2,4-bis-(4-methoxyphenyl)-1,3-dithio-2,4-diphosphetane-2,4-disulfide

Preparation M

(3S,4R,5R)-3-[1-(2,2,2-trichloroethoxycarbonyloxyethyl)]-4(0)-thioacetoxy azetidin-2-one

A solution of (3S,4R,5R)-3-[1-(2,2,2-trichloroethoxycarbonyloxyethyl)]-4-acetoxy azetidin-2-one (1 g) and Lawesson's reagent (1.4 g) in toluene (20 ml) is heated at 110°C. for 3 hours. The reaction mixture is then concentrated and chromatographed on silica gel to afford the title compound.

Preparation N

(3S,4R,5R)-3-[1-(2,2,2-trichloroethoxycarbonyloxyethyl)]-4-hydroxperoxy azetidin-2-one

A mixture of 30% hydrogen peroxide (0.3 ml) and 4N sodium hydroxide (0.7 ml) is added dropwise to a solution of (3S,4R,5R)-3-[1-(2,2,2-trichloroethoxycarbonyloxyethyl)]-4-acetoxy azetidin-2-one (1 g) in ethanol (20 ml). The mixture is allowed to stand for 24 hours, diluted with water and extracted with ethylacetate. The organic extract is concentrated under reduced pressure and chromatographed on silica gel to afford the title compound.

Preparation O

(3S,4R,5R)-3-[1-(2,2,2-trichloroethoxycarbonyloxyethyl)]-4-[(ethoxy) carbonothioyl oxy[-azetidin-2-one

A solution of (3S,4R,5R)-3-[1-(2,2,2-trichloroethoxycarbonyloxyethyl)]-4-hydroperoxy azetidin-2-one (1 g) and thiocarbonyl di-imidazole (0.6 g) in methylene chloride (10 ml) is treated with dimethylsulfide (0.4 g) for 3 hours and then with ethanol (0.5 ml) for 3 hours. The reaction mixture is then washed with water, concentrated and chromatographed on silica gel to afford the title compound.

Preparation P

(3S,4R,5R)-3-[1-(2,2,2-trichloroethoxycarbonyloxyethyl)]-4-[(ethylthio) carbonothioyl oxy]-azetidin-2-one

A solution of (3S,4R,5R)-3-[1-(2,2,2-trichloroethoxycarbonyloxyethyl)]-4-hydroperoxy azetidin-2-one (1 g), carbon disulfide (0.4 ml) and ethyl iodide (0.6 g) in benzene (10 ml) is stirred while adding a solution of dimethyl sulfide (0.2 ml) and triethylamine (0.4 ml). The reaction mixture is stirred for 3 hours and the title compound is isolated by chromatography on silica gel.

Preparation P

(3S,4R,5R)-3-[1-(2,2,2-trichloroethoxycarbonyloxyethyl)]-4- (ethylamino-thiocarbonyl-oxy)-azetidin-2-one

A solution of (3S,4R,5R)-3-[1-(2,2,2-trichloroethoxycarbonyloxyethyl)]-4-hydroperoxy azetidin-2-one (1 g) and thiocarbonyl-di-imidazole (0.6 g) in methylene chloride (10 ml) is treated with dimethylsulfide (0.4 g) for 3 hours and then with ethylamine (0.15 g) for 3 hours. The reaction mixture is then washed with water, concentrated under reduced pressure and chromatographed on silica gel to afford the title compound.

Preparation R

(3S,4R,5R)-3-[1-(2,2,2-trichloroethoxycarbonyloxyethyl)]-4-(ethylamino-thiocarbonyl methyl)-azetidin-2-one

A solution of (3S,4R,5R)-3-[1-(2,2,2-trichloroethoxycarbonyloxyethyl)]--4-carboxymethyl azetidin-2-one (1.0 g) and ethylamine (0.15 g) in ethylacetate (10 ml) is cooled to 0°C. and treated with dicyclohexylcarbodiimide (0.6 g). The mixture is allowed to stand at 0°C. and then filtered. The filtrate is evaporated to dryness and the residue is redissolved in toluene (20 ml) containing Lawesson's reagent (1.2 g). The solution is heated for 3 hours following which the title compound is isolated by chromatography on silica gel.

Preparation S

(3S,4R,5R)-3-[1-(2,2,2-trichloroethoxycarbonyloxyethyl)]-4-(ethoxythiocarbonyl-methyl)-azetidin-2-one

A solution of (3S,4R,5R)-3-[1-(2,2,2-trichloroethoxycarbonyloxyethyl)]-4-carbethoxymethyl-azetidin-2-one (1 g) and Lawesson's reagent (1.2 g) in toluene (20 ml) is heated for 3 hours, followed by chromatography on silica gel to afford the title compound.

Preparation T

(3S-4R,5R)-3-[1-(2,2,2-trichloroethoxycarbonyloxyethyl)]-4-(propan-2-thione)-azetidin-2-one

A solution of (3S,4R,5R)-3-[1-(2,2,2-trichloroethoxycarbonyloxyethyl)]-4-(propan-2-one)-azetidin-2-one (1.0 g) and Lawesson's reagent (1.2 g) in toluene (20 ml) is heated for 3 hours followed by chromatography on silica gel to afford the title compound.

Preparation U

(3S,4R,5R)-3-[1-(2,2,2-trichloroethoxycarbonyloxyethyl)]-4-{2'-(2''-[β-allyloxycarbonylamino]-ethylthio-thiocarbonyl)-ethyl}-azetidin-2-one

(3S,4R,5R)-3-[1-(2,2,2-trichloroethoxycarbonyloxyethyl)]-4-carboxyethyl-azetidin-2-one is subjected to the sequence of reactions described for Preparation L to afford the title compound.

Preparation V

(3S,4R,5R)-3-[1-(2,2,2-trichloroethoxycarbonyloxyethyl)]-4-(2'-[ethylamino thiocarbonyl]ethyl)-azetidin-2-one

(3S,4R,5R)-3-[1-(2,2,2-trichloroethoxycarbonyloxyethyl)]-4-carboxyethyl-azetidin-2-one is subjected to the sequence of reactions described for Preparation R to afford the title compound.

Preparation W

(3S,4R,5R)-3-[1-(2,2,2-trichloroethoxycarbonyloxeyethyl)]-4-(pentan-3'-thione)-azetidin-2-one

(3S,4R,5R)-3-[1-(2,2,2-trichloroethoxycarbonyloxyethyl)-4-(pentan-3'-one)-azetidin-2-one is reacted with Lawesson's reagent as described for Preparation T, to afford the title compound.

Preparation X

(3S,4R,5R)-3-[1-(2,2,2-trichloroethoxycarbonyloxyethyl)]-4-dithioacetoxy azetidin-2-one

A solution of (3S,4R,5R)-3-[1-(2,2,2-trichloroethoxycarbonyloxyethyl)]-4-acetoxy azetidin-2-one (1 g), dithioacetic acid (0.3 g) and sodium bicarbonate (0.25 g) in ethanol (10 ml) is stirred for 24 hours. The reaction mixture is diluted with water, extracted with ethylacetate and the extract is evaporated under reduced pressure to afford the title compound.

Example 3

Allyl (5R,6S,8R)-6-[1-(2,2,2-trichloroethoxycarbonyloxyethyl)]-2-methoxy-2-penem-3-carboxylate

The title compound of Preparation C is subjected to the N-acylation and cyclization process described for Example 2 to afford the title compound as colourless crystals, IR 5.56 μ.

NMR: δ 5.62 ppm (1H, d, J=1.8 cps)
3.9 ppm (1H, q, J=7; 1.8 cps)
4.02 ppm (3H, s).
MS: M± 459,461.

Example 4

Allyl (5R,6S,8R)-6-[1-(2,2,2-trichloroethoxycarbonyloxyethyl)]-2-propoxy-2-penem-3-carboxylate

The title compound of Preparation D is subjected to the N-acylation and cyclization process described for Example 2 to afford the title compound.as colourless crystals, M.p. 81°C.—82°C., IR 5.58 μ.

NMR: δ 5.58 ppm (1H, d, J=1.8 cps)
3.9 ppm (1H, q, J=8; 1.8 cps)
4.1 ppm (2H, t, J=7 cps).
MS: M± 487,489.

## Example 5

Allyl (5R,6S,8R)-6-[1-(2,2,2-trichloroethoxycarbonyloxyethyl)]-2-butoxy-2-penem-3-carboxylate

The title compound of Preparation E is subjected to the N-acylation and cyclization process of Example 2 to afford the title compound as a colour less oil, IR 5.58 μ.

NMR: 5.6 ppm (1H, d, J=1.8 cps)
3.9 ppm (1H, q, J=8; 1.8 cps)
4.2 ppm (2H, t, J=7 cps).
MS: m± 501,503.

## Example 6

Allyl (5R,6S,8R)-6-[1-(2,2,2-trichloroethoxycarbonyloxyethyl)]-2-(2′-allyloxycarbonylamino ethoxy)-2-penem-3-carboxylate

The title compound of Preparation F is subjected to the N-acylation and cyclization process of Example 2 to afford the title compound as crystalline solid m.p. 71°C.-76°C.

$[\alpha]_D$ + 59° (chloroform).
NMR: 5.59 ppm (1H, d, J=1.8 cps)
3.88 ppm (1H, q, J=8.8; 1.8 cps).
MS: M± 572,574.

## Example 7

Allyl-(5R,6S,8R,2′S)-2-([2′-allyloxycarbonyl-amino-2′-allyloxycarbonylethyl] thio)-6-(1-trichloroethoxycarbonyloxyethyl)-2-penem-3-carboxylate

A solution of (3S,4R,5R,2′S)-3-[1-(2,2,2-trichloroethoxycarbonyloxyethyl)]-4-[(2′-allyloxycarbonyl-amino-2′-allyloxycarbonylethyl thio)-carbonothioyl thio]-azetidin-2-one (24.7 g) in methylene chloride (200 ml) containing calcium carbonate (25 g) is cooled to 5°C. and treated with a solution of allyloxyoxalyl chloride (7.29 g) in methylene chloride (30 ml). The reaction mixture is stirred well while adding diisopropyl ethylamine (8.42 ml) in methylene chloride (30 ml) at a rate to maintain the reaction temperature below 7°C. The mixture is stirred for an additional 10 minutes, and then worked-up adding ice/water (100 ml) with vigorous stirring. The mixture is filtered and the organic layer of the filtrate is washed with ice/water (100 ml), dried in an ice-bath with sodium sulfate and diluted to 1500 ml with ethanol-free chloroform into a 2 litre 3-neck flask. The diluted solution is reflexed while adding a solution of triethyl phosphite (16.7 ml) in chloroform (50 ml) during 3½ hours using a syringe pump. The reaction mixture is refluxed under nitrogen overnight and then evaporated under reduced pressure. The residual oil is chromatographed on silica gel (250 g). Elution with 20% ETOAC/hexane affords the title compound (21.2 g) as a colourless oil.

$[\alpha]_D$ + 111.6° (chloroform).
IR (nujol) 5.58, 5.70, 5.80 μ.
NMR ($C_DCl_3$): 1.55 (d, 3H, J=7 cps)
3.45 (d, 2H, J=5 cps)
3.90 (dd, 1H, J=2 cps)
5.65 (d, 1H, J=2 cps).

## Example 8

Allyl (5R,6S,8R)-6-[1-(2,2,2-trichloroethoxycarbonyloxyethyl)]-2-ethoxy 1-cyclohexyl-1-azapen-2-em 3-carboxylate

The dithiocarbamate from Preparation H is reacted with allyloxyoxalyl chloride followed by triethylphosphite using the procedure described for Example 2 to afford the title compound.

## Example 9

Allyl (5R,6S,8R)-6-[1-(2,2,2-trichloroethoxycarbonyloxyethyl)]-2-ethylthio-cyclohexyl-1-azapen-2-em 3-carboxylate

The dithiocarbamate from Preparation I is subjected to the acylation and cyclization process decribed for Example 2 to afford the title compound.

## Example 10

Allyl (5R,6S,8R)-6-(1-(2,2,2-trichloroethoxycarbonyloxyethyl)]-1N,2N-diacetyl-2-amino-1-azapen-2-em 3-carboxylate

The thiourea from Preparation J is subjected to the acylation and cyclization process described for Example 2 to afford the title compound.

## Example 11

Allyl (5R,6S,8R)-6-[1-(2,2,2-trichloroethoxycarbonyloxyethyl)]-2-acetylamino 2-penem 3-carboxylate

The diothiocarbamate of Preparation K is subjected to the N-acylation and cyclization process

described for Example 2 to afford the title compound.

Example 12

Allyl (5R,6S,8R)-6-[1-(2,2,2-trichloroethoxycarbonyloxyethyl)]-2-[2'-)allyloxycarbonylamino)ethylthio]-1-carbapen-2-em 3-carboxylate

The dithio ester of Preparation L is subjected to the N-acylation and cyclization process described for Example 2 to afford the title compound.

Example 13

Sodium (5R,6S,8R)-6-(1-hydroxyethyl)-2-ethoxy-2-penem 3-carboxylate

A solution of allyl (5R,6S,8R)-6-[1-(2,2,2-trichloroethoxycarbonyloxyethyl)]-2-ethoxy-2-penem-3-carboxylate (0.2 g) in tetrahydrofuran (2 ml) containing water (0.5 ml) and acetic acid (1 ml) is cooled to −20°C. and stirred for 2½ hours with zinc dust (0.2 g). The solution is then filtered, the filtrate is diluted with ethylacetate, washed with brine, 10% sodium bicarbonate, dried and evaporated to afford allyl (5R,6S,8R)-6-(1-hydroxyethyl)-2-ethoxy-2-penem-3-carboxylate as an oil (132 mg) which is dissolved in methylene chloride (1 ml). The solution is then stirred with tetrakis (triphenylphosphine)palladium (30 mg) triphenylphosphine (30 mg) and 0.5M 2-ethyl-hexanoic acid sodium salt (0.8 ml). After 20 minutes the reaction mixture is extracted with cold water (5 × 2 ml) and the aqueous extract is lyophylised to afford the title compound.

NMR: δ ($D_2O$) 5.6 ppm (1H, d, J=1.8 cps)
4.22 ppm (2H, q, J=6 cps)
3.85 ppm (1H, q, J=6; 1.8 cps)

Example 14

Sodium (5R,6S,8R)-6-(1-hydroxyethyl)-2-ethoxy-1-cyclohexyl-1-azapen-2-em 3-carboxylate

The azapenem from Example 8 is deprotected using the procedure for Example 13 to afford the title compound.

Example 15

Sodium (5R,6S,8R)-6-(1-hydroxyethyl)-2-ethylthio-1-cyclohexyl 1-azapen-2-em 3-carboxylate

The azapenem from Example 9 is deprotected using the procedure described for Example 13 to afford the title compound.

Example 16

Sodium (5R,6S,8R)-6-(1-hydroxyethyl)-N,2N-diacetyl 2-amino 1-azapen-2-em 3-carboxylate

The azapenem from Example 10 is deprotected using the procedure described for Example 13 to afford the title compound.

Example 17

Sodium (5R,6S,8R)-6-(1-hydroxyethyl)-2-acetylamino-2-penem-3-carboxylate.

The penem from Example 11 is deprotected using the procedure described for Example 13 to afford the title compound.

Example 18

Allyl (5R,6S,8R)-6-(1-(2,2,2-trichloroethoxycarbonyloxyethyl)]-2-methyl-1-oxapen-2-em-3-carboxylate

The thioester of Preparation M is subjected to the N-acylation and cyclization process described for Example 2, to afford the title compound.

Example 19

Allyl (5R,6S,8R)-6-[1-(2,2,2-trichloroethoxycarbonyloxyethyl)]-2-ethoxy-1-oxapen-2-em-3-carboxylate

The compound of Preparation O is subjected to the N-acylation and cyclization process described for Example 2, to afford the title compound.

Example 20

Allyl (5R,6S,8R)-6-[1-(2,2,2-trichloroethoxycarbonyloxyethyl)]-ethylthio-1-oxapen-2-em-3-carboxylate

The dithioester of Preparation P is subject to the N-acylation and cyclization process described for Example 2, to afford the title compound.

Example 21

Allyl (5R,6S,8R)-6-[1-(2,2,2-trichloroethoxycarbonyloxyethyl)]-2-ethylamino-1-oxapen-2-em-3-carboxylate

The compound of Preparation Q is subjected to the N-acylation and cyclization process described for Example 2, to afford the title compound.

Example 22

Allyl (5R,6S,8R)-6-[1-(2,2,2-trichloroethoxycarbonyloxyethyl)-2-ethylamino-1-carbapen-2-em-3-carboxylate

The thioamide of Preparation R is subjected to the N-acylation and cyclization process described for Example 2, to afford the title compound.

Example 23

Allyl (6R,7S,9R)-7-[1-(2,2,2-trichloroethoxycarbonyloxyethyl)]-3-(2'allyloxycarbonylamino)ethylthio-1-carbaceph-3-em-4-carboxylate

The dithioester of Preparation U is subjected to the N-acylation and cyclization process described for Example 2, to afford the title compound.

Example 24

Allyl (6R,7S,9R)-7-[1-(2,2,2-trichloroethoxycarbonyloxyethyl)]-3-ethylamino-1-carbaceph-3-em-carboxylate

The compound of Preparation V is subjected to the N-acylation and cyclization process described for Example 2, to afford the title compound.

Example 25

Allyl (6R,7S,9R)-7-[1-(2,2,2-trichloroethoxycarbonyloxyethyl)]-3-ethyl-1-carbaceph-3-em-4-carboxylate

The thioketone of Preparation W is subjected to the N-acylation and cyclization process described for Example 2, to afford the title compound.

Example 26

Allyl (5R,6S,8R)-6-[1-(2,2,2-trichloroethoxycarbonyloxyethyl)]-2-ethoxy-1-carbapen-2-em-3-carboxylate

The thioester of Preparation S is subjected to the N-acylation and cyclization process described for Example 2, to afford the title compound.

Example 27

Allyl (5R,6S,8R)-6-[1-(2,2,2-trichloroethoxycarbonyloxyethyl)]-2-methyl-1-carbapen-2-em-3-carboxylate

The thioketone of Preparation T is subjected to the N-acylation and cyclization process described for Example 2, to afford the title compound.

Example 28

Allyl (5R,6S,8R)-6-[1-(2,2,2-trichloroethoxycarbonyloxyethyl)]-2-methyl-2-penem-3-carboxylate

The diothioester of Preparation X is subjected to the N-acylation and cyclization process described for Example 2, to afford the title compound.

Example 29

A. 3S,4R,5R-3-[1-(2,2,2-trichloroethoxycarbonyloxyethyl)]-4-(2-allyloxycarbonylethylene-thiocarbothioylthio)-azetidin-2-one

Dissolve 6.5 g of methyl (5R,6S,8R)-6-[1-(2,2,2-trichloroethoxycarbonyloxyethyl)-penicillanate in 100 ml of methylene chloride and cool to −20°C. Add a solution of carbon tetrachloride containing 34.5 mmoles of chlorine and allow the reaction to proceed for 30 minutes. Evaporate the reaction mixture. Add 25 ml of carbon tetrachloride and re-evaporate the solution to a residue. Dissolve the residue in 200 ml of methylene chloride and ozonize the solution at −70°C until a deep blue color is obtained. Add 10 ml of dimethyl sulfide and stir the reaction mixture for 30 minutes, then evaporate to a residue.

Dissolve the residue in 100 ml of methylene chloride and add to a thiocarbonate solution at −20°C which is prepared as follows: Dissolve 60 mmole (8.8 g) allyl 3-mercaptopropionate in 100 ml of ethanol and cool to −20-C. Add 45 ml of 1.0 molar aqueous potassium hydroxide, followed by 10 ml of carbon disulfide. Stir the reaction mixture for 30 minutes at −20°C. Add the methylene chloride solution of the chlorolactam prepared above. Stir the reaction mixture at −20°C for 45 minutes and then add to a two phase solvent mixture of water and ethyl ether. Wash the ether layer with a solution of sodium bicarbonate, then dry the ether solution over magnesium sulfate. Evaporate the ether solution to a residue and chromatograph on silica gel using an increasing concentration of ether in methylene chloride as the eluant. Combine and evaporate like fractions to obtain thereby a thick yellow oil (3.8 gm). PMR ($CDCl_3$) 1.53 (d, J=7Hz, 2H), 2.82 (m, 2H), 3.46 (dd, J=2.5 and 8Hz, 1H), 3.65 (m, 2H), 4.64 (d, J=6Hz, 2H), 4.80 (s, 2H), 5.1—5.5 (m 3H), 5.68 (3, J=2.5Hz, 1H), 5.6—6.2 (m, 1H and 6.8 (br, s, 1H, exch. by $D_20$).

B. Allyl (5R,6S,8R)-2-(2-allyloxycarbonylethylenethio)-6-[1,(2,2,2-trichloroethoxycarbonyloxyethyl)]-penem-3-carboxylate

Dissolve 35.5 gms of the product from Step A in 400 ml of methylene chloride, cool to 0—5°C and add 12.4 g of allyloxalyl chloride. Add dropwide with stirring 10.8 g of diisopropylethylamine. Stir for 10—20 minutes at 0—5°C, then treat with ice cold 1N sulfuric acid. Separate the layers, wash the organic layer with water and dry over magnesium sulfate. Filter and dilute the filtrate with 350 ml ethanol free chloroform. Reflux the resulting solution under nitrogen. Add over a three-hour interval 23 g of triethylphosphite, evaporate the mixture to a residue and chromatograph on silica gel using dichloromethanehexane, then

dichloromethane and finally 2% ethyl ether in dichloromethane as the eluant. Combine like fractions and evaporate to obtain thereby the product of this step as a yellow oil. Yield 16.0 g. IR, max ($CH_2Cl_2$) 1795, 1755, 1730, 1695 cm—1 PMR ($CDCl_3$) 1.37 (d, J=7Hz, 3h), 2.6 (m, 2H), 3.1 (m, 2H), 4.77 (dd, J=8 and 2 Hz, 1H), 4.4—4.7 (m, 4H), 4.64 (s, 2H), 4.9—5.4 (d, J=2Hz, 1H), 5.54 (d, J=2H, 1H) and 5.6—6.1 (m, 2H).

C. Allyl 5R,6S,8R-6-(1-hydroxyethyl)-2-(allyloxycarbonylethylenethio)-2-penem-3-carboxylate

Dissolve 1.6 g of the product prepared according to the process of steps A and B, in 15 ml of tetrahydrofuran, 1.5 ml of water and 1.5 ml of acetic acid at 0—5°C with stirring. Add 2.0 g of zinc dust until thin layer chromatography indicates only a trace of starting material. Filter the reaction mixture, wash solids with ethylacetate, combine the organic solvents and wash successively with 10% aqueous tartaric acid, water and with aqueous sodium bicarbonate solution. Dry the solvent phase over magnesium sulfate and concentrate to a residue. Crystallize the residue from ether-hexane to obtain thereby the product of this step as fine white needles MP 95—96°C. IR ($CH_2Cl_2$) max 3500, 1790, 1725 and 1695 cm—1 PMR ($CDCl_3$): 1.35 (3, J=7HZ, 3H), 1.86 (d, J=7HZ, 1H, exch. by $D_2O$), 2.75 (m, 2H), 3.20 (m, 2H), 3.73 (33, J=8 and 2Hz, 1H), 4.24 (m, 1H), 4.55—4.8 (m, 4H, 5.1—5.6 (m, 4H), 5.67 (d, J=2HZ, 1H) and 5.7—6.15 (m 2H).

D. Disodium (5R,6S,8R)-6-(1-hydroxyethyl)-2-(2-carboxyethylenethio)penem-3-carboxylate

Disolve 6.4 g of the product prepared according to Steps A—C in 190 ml of methylene chloride and add 5.32 g of sodium 2-ethylhexanoate in 190 ml of ethylacetate. Add a mixture of 0.46 gm of triphenylphosphine and 0.46 g of tetrakis (triphenylphosphine) palladium. Stir for 1.5 hours and centrifuge. Wash the precitate with ethylacetate and dry at high vacuum to give pale brown powder. Yield 6.2 g [α]26 D($H_2O$) = + 153.7. PMR($D_2O$): 1.36 (d, J=7Hz, 2H), 2.6 (m, 2H), 3.2 (m, 2H), 3.93 (dd, J=8 and 2Hz, 1H), 4.28 (m, 1H) and 5.71 (d, J=2Hz, 1H).

Example 30

A. (3S,4R,5R)-3-(2,2,2-trichloroeth-1-oxycarbonyloxyethyl)-4-(2-hydroxyethylthiocarbothioyl)-azetidin-2-one

Dissolve 10 g. of Methyl-(5R,6S,8R)-2-(2,2-dimethyl)-6-(2,2,2-trichloroeth-1-oxycarbonyloxyethyl)-penam-3-carboxylate in 150 ml. of methylene chloride at 0°—5°C., add 7.36 ml. of sulfuryl chloride and stir for one hour at room temperature. Pour the reaction mixture into an excess of aqueous sodium bicarbonate with stirring. Separate the two liquid phases, dry the organic phase and evaporate to a residue. Dissolve the residue in 100 ml. of methylene chloride and treat with ozone at −78°C. until the blue color persists, then add 5 ml. of dimethyl sulfide to the reaction mixture at room temperature for one hour, then add to a stirred ice cold trithiocarbonate solution prepared from 10 ml. of betamercaptioethanol and 6 g. of potassium hydroxide in 200 ml. of 50% aqueous ethanol cooled to 0°C. and treated with 28 ml. of carbon disulfide. Allow the mixture of the chlorolactam and the trithiocarbonate solution to react at 0°C. for 45 minutes with stirring, then dilute with water. Extract the reaction mixture with methylene chloride, wash with aqueous sodium bicarbonate, dry over magnesium sulfate and evaporate to a residue. Chromatograph the residue on silica gel, eluting with an increasing concentration of ethyl ether in methylene chloride to 30%. Combine like fractions containing the title compound as determined by thin layer chromatography and evaporate to obtain, thereby, the product of this step as a light yellow oil.

Yield — 8.1 g.

I.R. ($CH_2Cl_2$) 3550, 1770, 1750 cm—1.

B. (3S,4R,5R)-3-(2,2,2-trichloroeth-1-oxycarbonyloxyethyl)-4-[2-(t-butyldimethylsilyloxy)ethylthiocarbothiolthio]-azetidin-2-one

Dissolve 7.07 g. of the product from Step A in a mixture 50 ml. of methylene chloride and 1.43 ml. of pyridine, 2.64 g. of t-butylchloridimethylsilane and 0.1 g. of imidazole. Stir the solution at room temperature for two days, wash with water and evaporate the residue. Chromatograph the residue in silica gel using dichloromethane:hexane and then methylene chloride with increasing concentrations of ethyl ether. Combine like fractions containing the title compound as determined by thin layer chromatography and evaporate to obtain thereby, the title compound as a light yellow oil.

Yield — 8.4 g.

I.R. 3400, 1770, and 1750 cm—1.

C. Allyl-(5R,6S,8R)-2-[2′-(t-butyldimethylsilyloxy)ethylthio]-6-(2,2,2-trichloroeth-1-oxycarbonyloxy)-penem-3-carboxylate

Dissolve 8.4 g. of the product of Step B in 50 ml. of methylene chloride containing 2.69 g. of allyl oxalyl chloride and stir at 0°—5°C. while adding 2.32 g. of diisopropylethylamine in 15 ml. of methylene chloride dropwise. Stir the reaction mixture for an additional half-hour at 0—5°C., wash with water, with dilute hydrochloric acid and with dilute aqueous sodium bicarbonate. Dry the organic solvent phase over magnesium sulfate, filter and evaporate to a residue. Dissolve the residue in 100 ml. of ethanol-free chloroform, add 1.0 g. of calcium carbonate during the addition of 5 g. of triethyl phosphite over a 3-hour interval. Reflux the solution for an additional 18 hours, cool and chromatograph on silica gel eluting with methylene chloride:hexane, methylene chloride and finally with 1% ethyl ether in methylene chloride. Combine like fractions containing the title compound as determined by thing layer chromatography to

obtain thereby the title compound as a yellowish oil. 'H NMR ($CDCl_3$): 0.10 (s, 6), 0.92 (s, 9), 1.54 (d, 3, J=7), 3.07 ( , 2), 3.84 (m, 3), 4.76 (m, 2), 4.79 (s, 2),5.1—5.6 (m, 3), 5.64 (d, 1, J—2.5) and 5.7—6.2 (m, 1).

D. Allyl-(5R,6S,8R)-2-(2-hydroxyethylthio)-6-(2,2,2-trichloroeth-1-oxy-carbonyloxy)-penem-3-carboxylate

Dissolve 4.46 g. of the product of Step C in a mixture of 32 ml. of tetrahydrofuran, 4 ml. of water and 4 ml. of acetic acid. Stir the solution for 18 hours at room temperature with 2.4 g. of tetra-n-butylammonium flouride. Pour the reaction mixture into a two-phase solvent system consisting of methylene chloride and water, with stirring. Wash the organic phase with aqueous sodium bicarbonate. Dry the organic phase over magnesium sulfate, filter and evaporate to a residue. Chromatograph the residue on silica gel using ethyl ether:methylene chloride as the eluant. Combine like fractions containing the title compound as determined by thin layer chromatography and evaporate to obtain thereby the title compound of this example as a yellowish oil.

Yield — 2.9 g.

'H NMR ($CDCl_3$): 1.49 (3, 3, J=7), 2.17 (m, 1, exch by $D_2O$), 3.12 (m, Z), 3.704.0 (m, 3), 4.72 (m, 2), 4.76 (s, 2), 5.1—5.6 (m, 3), 5.67 (d, 2, J=2.5) and 5.7—6.2 (m, 1).

E. Allyl-(5R,6S,8R)-2-(2-hydroxyethylthio)-6-1-hydroxyethyl)-penem-3-carboxylate

Dissolve 1.4 g. of the product of Step D in a mixture consisting of 1.5 ml. of acetic acid, 1.5 ml. of water and 15 ml. of tetrahydrofuran. Add 1.25 g. of zinc dust and stir the mixture at 0°—5°C. Monitor the reaction by thin layer chromatography until the starting material is substantially all converted (approximately 1 hour). Filter, wash the solids with ethyl acetate and the filtrate with saturated sodium bicarbonate solution. Dry the filtrate over magnesium sulfate, filter and evaporate the filtrate to a residue. Crystallize the residue from the ethyl ether:methylene chloride to obtain the title compound of this step as white needles.

Yield — 0.5 g. M.P. 83—85°C.

'H NMR ($CDCl_3$): 1.37 (d, e, J=7), 2.5—2.7 (n, Z, exch by $D_2O$), 3.14 (m, 2), 3.73 (dd, 1, JJ=8.25), 3.84 (q, 2, J=7), 4.23 (m, 1), 4.76 (m, 2), 5.2—5.6 (m, 2), 5.67 (d, 1, J=2.5) and 5.8—6.25 (m, 1).

F. Sodium-(5R,6S,8R)-2-(2-hydroxyethylthio)-6-(1-hydroxyethyl)-penem-3-carboxylate

Dissolve 200 mg of the product of Step E in 4 ml of methylene chloride, dissolve 0.105 g of sodium 2-ethylhexanoate in 2 ml of ethyl acetate, mix the two solutions. Add to the resulting solution 14 mg of triphenylphosphate, 14 mg of tetrakis (triphenylphosphine) palladium and stir the mixture at room temperature under nitrogen for 1 hour. Dilute the reaction mixture with 20 ml of ethyl acetate and extract with water (3 × 10 ml).

Pass a stream of nitrogen through the aqueous extract to remove residual organic solvents, filter and lyophilize the aqueous layer to obtain thereby the title compound as a pale brown powder.

Yield — 190 mg.

'H NMR ($D_2O$): 1.24 (d, 3, J=7), 3.0 (m, 2), 3.77 (t, 2, J=7), 3.63 (dd, 1, J=6, 2), 4.15 (m, 1), and 5.60 (d, 1, J=2).

Preparation Y

(3S,4R,5R)-(2-methoxy-1,2-dioxo ethyl)-3-(2,2,2-trichloroeth-1-oxycarbonyloxyethyl)-4-chloro-azetidin-2-one

a) A solution of methyl (5R,6S,8R)-6-(2,2,2-trichloroeth-1-oxycarbonyloxyethyl)-penicillanate (5.0 g) in $CH_2Cl_2$ (50 ml) at 0°C to 5°C. is treated with sulfuryl chloride (6.2 g). After stirring at 0° to 5°C for 15 minutes and thereafter at room temperature for 1 hour, the mixture is added to ice-cooled sodium bicarbonate solution with stirring. When $CO_2$ evolution has ceased, the organic phase is dried over magnesium sulphate and evaporated. The residue is dissolved in dichloromethane (50 ml), stirred and cooled to −70°C. and ozone introduced whereupon the reaction mixture changes to a permanent deep blue colour. The excess ozone is removed by a brief stream of nitrogen; dimethyl sulfide (5 ml) is then added to the solution kept at room temperature for 2 hours, to afford the title product.

b) (3S,4R,5R)-3-(2,2,2-trichloroeth-1-oxycarbonyloxyethyl)-4-(t-butylthio carbothioylthio)-azetidin-2-one

Thiocarbonate solution is prepared at 0°C. in known manner from 1M aqueous KOH solution (43 ml) with t-butyl thiol (4 g) in ethanol (70 ml) followed by $CS_2$ (10 ml). After stirring for 10 minutes at 0—5°C., the solution obtained in part a) is added and the mixture stirred for 45 minutes at 0°C. The mixture is worked up and the crude product chromatographed on silica, eluting with $CH_2Cl_2$-hexane, then $CH_2Cl_2$ and finally with 2% ether in $CH_2Cl_2$. Pure fractions are evaporated to give the title product as a deep yellow oil.

pmr ($CDCl_3$), δ 1.57 (3, 3, J=7); 1.66 (2, 9); 3.49 (33, 1, J=2.5 and 9); 4.86 (s, 2); 5.36 (m, 1); 5.67 (s, 1, J=2.5) and 6.9 (br.s, 1).

Preparation Z

(3S,4R,5R)-3-(2,2,2-trichloroeth-1-oxycarbonylethyl)-4-(isopropylthiocarbothioylthio)-azetidin-2-one

Follow the procedure of Preparation Y but using an equivalent amount of propane-2-thiol in place of the t-butyl thiol in step (b) to obtain the title product as a yellow oil.

pmr ($CDCl_3$)δ: 1.48 (d, 6, J=7); 1.53 (d, 3, J=7); 3.46 (33, 1, J=2.5 and 9); 4.19 (m, 1); 4.81 (s, 2); 5.27 (m, 1); 5.67 (d, 1, J=2.5) and 7.0 (br.s, 1).

Preparation Z2

(3S,4R,5R)-3-(2,2,2-trichloroeth-1-oxycarbonyloxyethyl)-4-(3-allyloxycarbonyl-1-propylthiocarbothioylthio)-azetidin-2-one

Following the procedure of Preparation Y but substituting an equivalent amount of allyl-3-mercaptobutyrate for the t-butyl thiol in step (b), to obtain the title product as a yellow oil.

pmr ($CDCl_3$)δ: 1.53 (d, 3, J=7); 2.10 (m, 2) 2.53 (m, 2); 3.47 (m, 3), 4.62 (m, 2); 4.85 (s, 2), 5.2—5.5 (m, 3); 5.68 (d, 1, J=2.5); 5.8–6.2 (m, 1) and 7.1 (br.s, 1).

Example 31

A. i) allyl (5R,6S,8R)-2-(t-butylthio)-6-(2,2,2-trichloroethoxycarbonyloxy-ethyl)-penem-3-carboxylate

22 g of (3S,4R,5R)-1-(2-methoxy-1,2-dioxoethyl)-3-(2,2,2-trichloroeth-1-oxycarbonyloxyethyl)-4-chloroazetidin-2-one is stirred at 0 to 5°C in $CH_2Cl_2$ (40 ml) with allyloxyoxalyl chloride (1.3 g). Diisopropyl ethyl amine (1.1 g) is added dropwise to the reaction solution in 5 ml $CH_2Cl_2$ and the mixture stirred at 0 to 5°C for 15 minutes, washed twice with 50 ml water, dried and filtered.

The resulting solution is diluted with ethanol-free chloroform (50 ml) and heated at reflux with calcium carbonate (0.5 g) during the addition, over 2½ hours, of a solution of 2 g triethyl phosphite in 10 ml $CHCl_3$. After refluxing for 18 hours, the solution is filtered and evaporated and the residue chromatographed on silica gel, eluting with $CH_2Cl_2$-hexane followed by $CH_2CL_2$. Pure fractions were evaporated to give the title product as a pale yellow oil.

IR ($CH_2Cl_2$) ν 1790, 1750 and 1690 $cm^{-1}$.

ii) Allyl (5R,6S,8R)-2-(isoproylthio)-6-(2,2,2-trichloroeth-1-oxycarbonyloxyethyl)-penem-3-carboxylate

Following the procedure of part A(i) using the (3S,4R,5R)-3-(2,2,2-trichloroeth-1-oxycarbonyloxyethyl)-4-(isopropylthio-carbothioylthio)-azetidin-2-one from Preparation Z to obtain the title product, after chromatography and crystallisation from ether, as needles m.p. 60°—62°C. $[\alpha]^D_{26}$+206.1° ($CHCl_3$).

Analysis: Calc. for $C_{17}H_{21}NO_6Cl_3S_2$: C, 40.37; H, 4.18) N, 2.77; Cl, 21.03. Found C, 40.42; H, 3.85; N, 2.72; Cl, 21.05.

iii) Allyl (5R,6S,8R)-2-(3-allyloxycarbonyl-1-propylthio)-6-(2,2,2-trichoroeth-1-oxycarbonyloxyethyl)-penem-3-carboxylate

Follow the procedure of part A(i) using the (3S,4R,5R)-3-(1-trichloroethoxy carbonyloxyethyl)-4-(3-alkyloxycarbonyl-1-propylthiocarbothioylthio)-azetidin-2-one from Preparation Z2 to obtain the title product as a pale yellow oil.

IR ($CH_2Cl_2$) ν 1790, 1760, 1735 and 1695 $cm^{-1}$.

pmr ($CDCl_3$) δ: 1.53 (d, 3, J=7), 2.05 (m, 2); 2.48 (t, 2, J=7), 3.00 (t, 2, J=7); 3.89 (dd, 1, J=1.5 and 9); 4.5—4.8 (m, 4); 4.76 (s, 1); 5.1—5.5 (m, 5); 5.65 (d, 1, J=2.5) and 5.7—6.2 (m, 2).

B. (i) Allyl (5R,6S,8R)-2-(t-butylthio)-6-(1-hydroxyethyl)-penem-3-carboxylate

A mixture of allyl (5R,6S,8R)-2-(t-butylthio)-6-(2,2,2-trichloroeth-1-oxycarbonyloxyethyl)-penem-3-carboxylate (0.5 g), zinc dust (0.5 g), tetrahydrofuran (5 ml), water (0.5 ml) and acetic acid (0.5 ml) is stirred at 0 to 5°C for half an hour then at room temperature until no starting material remains, the mixture is worked up in ethylacetate $-H_2O$, washed with aqueous sodium bicarbonate, dried and evaporated and the product isolated on a preparative silica gel thin layer chromatography plate eluting with 5% ether-$CH_2Cl_2$. The product is obtained as a pale yellow oil; IR ($CH_2Cl_2$) ν max 3450, 1790 and 1690 $cm^{-1}$.

pmr ($CDCl_3$) δ: 1.33 (d, 3, J=7); 1.50 (s, 9); 2.75 (br.s, 1); 3.73 (dd, 1, J=1 and 7.5); 4.22 (m, 1); 4.70 (m, 1); 5.1—5.5 (m, 3), 5.58 (d, 1, J=1) and 5.8—6.2 (m, 1).

(ii) Allyl (5R, 6S,8R)-2-(isopropylthio)-6-(1-hydroxyethyl)penem-3-carboxylate

Follow the procedure in part B(i) using allyl (5R,6S,8R)-2-)isopropylthio)-6-(2,2,2-trichloroeth-1-oxycarbonyloxyethyl)-penem-3-carboxylate to obtain the title product in crude forms, which after workup and recrystallisation from ether-hexane is obtained as fibrous, white needles, mp 70—71°C. pmr spectrum ($CDCl_3$): 1.3—1.6 (m, 9); 2.9 (br.s, 1); 3.75 (dd, 1, J=8 and 1.5); 4.3 (m, 1); 4.75 (m, 2); 5.2—5.6 (m, 2); 5.70 (d, 1, J=1.5) and 5.8—6.2 (m, 1).

(iii) Allyl (5R-6S,iR)-2-(3-allyloxycarbonyl-1-propylthio)-6-(1-hydroxyethyl)-penem-3-carboxylate

Follow the prodcedure in part B(i) using allyl (5R,6S,8R)-2-(3-allyloxycarbonyl-1-propylthio)-6-(2,2,2-trichloroeth-1-oxycarbonyloxyethyl)penem-3-carboxylate, obtain the product, after chromatographic purification, as a pale yellow oil. pmr ($CDCl_3$) δ: 1.37 (d, 3, J=7); 2.2 (m, 2); 2.44 (br.s, 1); 2.53 (t, 2, J=7); 3.04 (t, 2, J=7); 3.75 (dd, 1, J=1.5 and 8); 4.27 (m, l); 4.55—4.85 (m, 4) 5.15—5.6 (m, 4); 5.69 (d, 1, J=1.5) and 5.8—6.2 (m, 2).

C. (i) Sodium (5R,6S,8R)-2-(t-butylthio)-6-(1-hydroxyethyl)-penem-3-carboxylate

0.07 of allyl (5R,6S,8R)-2-(t-butylthio)-6-(1-hydroxyethyl)-penem-3-carboxylate in $CH_2Cl_2$ (2 ml) is stirred under nitrogen with 0.45 ml of a 0.5 M solution of sodium-2-ethylhexanoate in ethylacetate. Pd $(PPh_3)_4$ (0.01 g) and $PPh_3$ (0.01 g) are added to the resulting solution and the mixture stirred at room

temperature until no starting material remains (0.5 hr). Ether (20 ml) is added, and the product is extracted twice into 10 ml water. The combined aqueous extracts are extracted with 10 ml ethylacetate, treated with a stream of nitrogen to remove dissolved organic solvents, and lyophilised to afford the title product as a pale yellow powder. IR (nujol) 1785 and 1600 $cm^{-1}$.

(ii) Sodium (5R,6S,8R))-2-(isopropylthio)-6-(1-hydroxyethyl)-penem-3-carboxylate

Follow the procedure of part C(i) using allyl (5R,6S,8R)-2-isopropylthio)-6-(1-hydroxy ethyl)-penem-3-carboxylate to obtain the title product after lyophilisation as a cream coloured hygroscopic powder. IR (nujol) vmax 1780 and 1610 $cm^{-1}$.

(iii) di-Sodium (5R,6S,8R)-2-(3-carboxyl-1-propylthio)-6-(1-hydroxyethyl)-penem-3-carboxylate

Follow the procedure of part C(i) using allyl (5R,6S,8R)-2-(3-allylcarbonyl-1-propylthio)-6-(1-hydroxyethyl)-penem-3-carboxylate but using double the amount of sodium-2-ethyl hexanoate. After lyophilisation the title product is obtained as pale brown powder. pmr ($D_2O$) δ: 1.30 (d, 3, J=7); 1.92 (m, 2); 2.17 (m, 2); 2.92 (m, 2); 2.92 (m, 2); 3.90 (dd, 1, J=1.5 and 8); 4.25 (1, 1, J=8) and 5.68 (d, 1, J=1.5).

**Claims for the Contracting States: BE DE FR GB NL**

1. A process for the preparation of a compound of the following general formula (I)

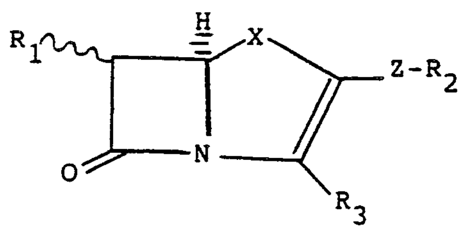

I

in which

$R_1$ is hydrogen, $C_1$—$C_6$ alkyl, acylamino or

$$R_4—\underset{}{CH}(OR_5)—,$$

wherein $R_4$ is hydrogen, $C_1$—$C_6$ alkyl, aryl, phenyl substituted by $C_1$—$C_6$ alkyl, by $C_1$—$C_6$ alkoxy or by halogen, or heteroaryl, said heteroaryl being an aryl group having a heteroatom in the ring and optionally having 1 to 3 $C_1$—$C_6$ alkyl substituents, and $R_5$ is hydrogen or an hydroxy protecting group;

$R_2$ is $C_1$—$C_6$ alkyl, aryl, phenyl substituted by $C_1$—$C_6$ alkyl, by $C_1$—$C_6$ alkoxy or by halogen, $C_1$—$C_6$ alkyl substituted by one or more aryl groups, aminoalkyl, *N*-protected aminoalkyl, hydroxyalkyl, O-protected hydroxyalkyl, an optionally esterfied, optionally N-protected α-amino acid residue or an optionally esterfied carboxyalkyl group;

$R_3$ is nitrile, tetrazolyl or —$COOR_6$, wherein $R_6$ is hydrogen, -Alk-$C(Hal)_3$ (in which Hal is halogen and Alk represents a $C_1$—$C_6$ alkylene radical), $C_1$—$C_6$ alkyl, aryl, phenyl substituted by $C_1$—$C_6$ alkyl, by $C_1$—$C_6$ alkoxy or by halogen, an allylic group, an ester group which can be metabolically removed *in vivo,* or a carboxy protecting group;

X and Z are independently sulfur, oxygen, —$CH_2$—, —$CH_2$—$CH_2$, or the radical =$NR_7$ in which $R_7$ is hydrogen, carboxylic acid acyl, $C_1$—$C_6$ alkyl, cycloalkyl containing 3 to 6 carbon atoms, aryl, phenyl substituted by $C_1$—$C_6$ alkyl, by $C_1$—$C_6$ alkoxy or by halogen, or an *N*-protecting group; or a pharmaceutically acceptable salt thereof; characterised by reacting an appropriate compound of the following general formula (II)

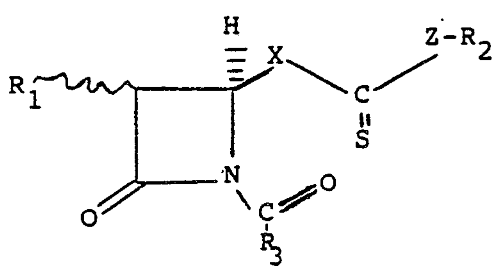

II

in which $R_1$, $R_2$, $R_3$, X and Z are as defined above wherein $R_5$ is a hydroxy protecting group, $R_7$ is not

hydrogen, any carboxy group in substituent $R_2$ is esterified, and $R_6$ is not hydrogen; with an organo-substituted derivative of phosphorous acid, and if required or desired, subjecting the resulting compound, prior to or subsequent to isolation and any separation into its stereochemical isomers, if a mixture of isomers of compounds of formula (II) was subjected to the foregoing reaction, to one or more of the following operations:

(a) removal of one or more protecting group;
(b) conversion of an appropriate function into a free acid;
(c) conversion of an appropriate function into a pharmaceutically acceptable salt; and
(d) conversion of an appropriate function into a metabolisable ester group;

provided that when $R_1$ is hydrogen, $C_1$—$C_6$ alkyl, hydroxyalkyl, acyloxyalkyl, alkylsulfonyloxyalkyl, aryl sulphonyloxyalkyl or trialkylsilyloxyalkyl, X and Z are both sulphur, $R_2$ is aminoalkyl, or N-protected aminoalkyl in which $R_2$-group the alkyl moiety is branched, and $R_3$ is —$COOR_6$, then the organo-substituted derivative cannot be a phosphorous acid triester or a phosphorous acid triamide.

2. A method according to claim 1, characterised in that the organo-substituted derivative of phosphorous acid is an acyclic trialkylphosphite.

3. A process according to claim 2, characterised in that two molar equivalents of trialkylphosphite are used per mole of the compound of formula (II), and the reaction is performed at a temperature of 40°C to 60°C.

4. A process according to any one of Claims 1 to 3, characterised by using a compound of formula (II) which has been prepared by the reaction of a compound of the following general formula (III)

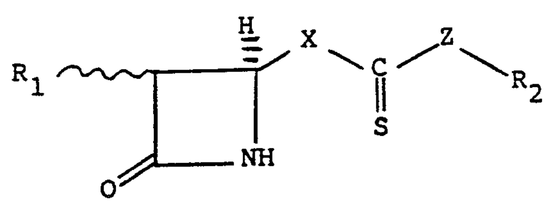

III

in which $R_1$, $R_2$, X and Z are as defined in Claim 1 with respect to formula (II), with a reactive derivative of an acid of the following general formula (IV)

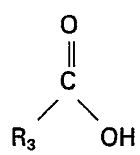

IV

in which $R_3$ is as defined in claim 1, in an inert solvent, in the presence of an organic base, and, when the reactive derivative is an acid halide, optionally an hydrogen halide binding agent.

5. A process according to claim 4, characterised by mixing in said inert solvent the compound of formula (III) and (per mole of the compound of formula (III)) approximately one molar equivalent of each of a halide derivative of the acid of formula (IV) and a tertiary amine, and at least one molar equivalent of a hydrogen halide binding agent.

6. A process according to claim 4 or 5, chacterised in that the acid halide of formula (IV) is allyloxyoxalylchloride or preferably chloroallyloxy oxalylchloride, the tertiary amine is di-isopropylethylamine, and the binding agent is calcium carbonate.

7. A process according to any one of the preceding claims, characterised in that a compound of formula (I), in which X and Z are both S, $R_1$ is

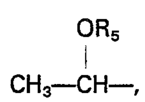

$R_2$ is $C_1$—$C_6$ alkyl, and $R_3$ is —$COOR_6$ in which $R_6$ is allyl or preferably chloroallyl is produced.

8. A method of preparing a pharmaceutical composition which comprises:

a) preparation of a pharmaceutically active compound of formula (I) according to any one of Claims 1 to 7, and

b) mixing the compound so prepared with a pharmaceutically acceptable carrier or excipient.

**Claims for the Contracting States: AT CH IT LI SE**

1. A process for the preparation of a compound of the following general formula (I)

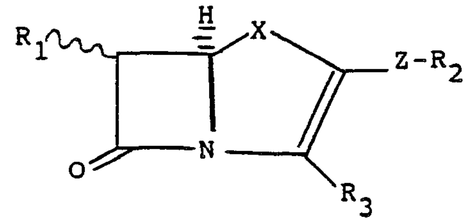

I

in which

$R_1$ is hydrogen, $C_1$—$C_6$ alkyl, acylamino or

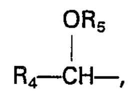

wherein $R_4$ is hydrogen, $C_1$—$C_6$ alkyl, aryl, phenyl substituted by $C_1$—$C_6$ alkyl, by $C_1$—$C_6$ alkoxy or by halogen, or heteroaryl, said heteroaryl being an aryl group having a heteroatom in the ring and optionally having 1 to 3 $C_1$—$C_6$ alkyl substituents, and $R_5$ is hydrogen or an hydroxy protecting group;

$R_2$ is $C_1$—$C_6$ alkyl, aryl, phenyl substituted by $C_1$—$C_6$ alkyl, by $C_1$—$C_6$ alkoxy or by halogen, $C_1$—$C_6$ alkyl substituted by one or more aryl groups, aminoalkyl, *N*-protected aminoalkyl, hydroxyalkyl, O-protected hydroxyalkyl an optionally esterfied, optionally N-protected α-amino acid residue or an optionally esterfied carboxyalkyl group;

$R_3$ is nitrile, tetrazolyl or —$COOR_6$, wherein $R_6$ is hydrogen, -Alk-C(Hal)$_3$ (in which Hal is halogen and Alk represents a $C_1$—$C_6$ alkylene radical), $C_1$—$C_6$ alkyl, aryl, phenyl substituted by $C_1$—$C_6$ alkyl, by $C_1$—$C_6$ alkoxy or by halogen, an allylic group, an ester group which can be metabolically removed *in vivo,* or a carboxy protecting group;

X and Z are independently sulfur, oxygen, —$CH_2$, —$CH_2$—$CH_2$, or the radical $=NR_7$ in which $R_7$ is hydrogen, carboxylic acid acyl, $C_1$—$C_6$ alkyl, cycloalkyl containing 3 to 6 carbon atoms, aryl, phenyl substituted by $C_1$—$C_6$ alkyl, by $C_1$—$C_6$ alkoxy or by halogen, or an *N*-protecting group; or a pharmaceutically acceptable salt thereof; characterised by reacting an appropriate compound of the following general formula (II)

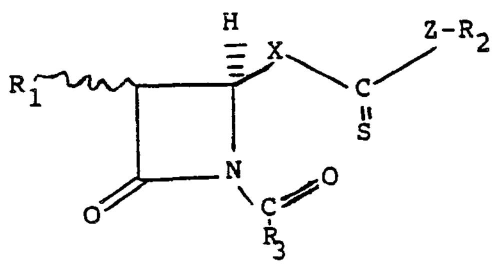

II

in which $R_1$, $R_2$, $R_3$, X and Z are as defined above wherein $R_5$ is a hydroxy protecting group, $R_7$ is not hydrogen, any carboxy group in substituent $R_2$ is esterified, and $R_6$ is not hydrogen; with an organo-substituted derivative of phosphorous acid, and if required or desired, subjecting the resulting compound, prior to or subsequent to isolation and any separation into its stereochemical isomers, if a mixture of isomers of compounds of formula (II) was subjected to the foregoing reaction, to one or more of the following operations:

(a) removal of one or more protecting group;
(b) conversion of an appropriate function into a free acid;
(c) conversion of an appropriate function into a pharmaceutically acceptable salt; and
(d) conversion of an appropriate function into a metabolisable ester group.

2. A method according to claim 1, characterised in that the organo-substituted derivative of phosphorous acid is an acyclic trialkylphosphite.

3. A process according to claim 2, characterised in that two molar equivalents of trialkylphosphite are used per mole of the compound of formula (II), and the reaction is performed at a temperature of 40°C to 60°C.

4. A process according to any one of Claims 1 to 3, characterised by using a compound of formula (II) which has been prepared by the reaction of a compound of the following general formula (III)

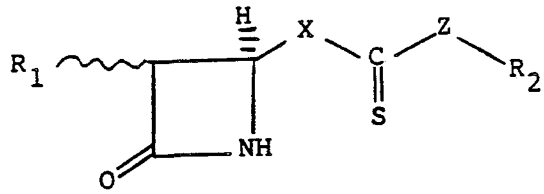

III

in which $R_1$, $R_2$, X and Z are as defined in Claim 1 with respect to formula (II), with a reactive derivative of an acid of the following general formula (IV)

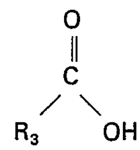

IV

in which $R_3$ is as defined in claim 1, in an inert solvent, in the presence of an organic base, and, when the reactive derivative is an acid halide, optionally an hydrogen halide binding agent.

5. A process according to claim 4, characterised by mixing in said inert solvent the compound of formula (III) and (per mole of the compound of forumla (III)) approximately one molar equivalent of each of a halide derivative of the acid of formula (IV) and a tertiary amine, and at least one molar equivalent of a hydrogen halide binding agent.

6. A process according to claim 4 or 5, characterised in that the acid halide of formula (IV) is allyloxyoxalylchloride or preferably chloroallyloxy oxalylchloride, the tertiary amine is di-isopropylethylamine, and the binding agent is calcium carbonate.

7. A process according to any one of the preceding claims, characterised in that a compound of formula (I), in which X and Z are both S, $R_1$ is

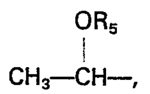

$R_2$ is $C_1$—$C_6$ alkyl, and $R_3$ is —$COOR_6$ in which $R_6$ is allyl or preferably chloroallyl is produced.

8. A method of preparing a pharmaceutical composition which comprises:

a) preparation of a pharmaceutically active compound of formula (I) according to any one of Claims 1 to 7, and

b) mixing the compound so prepared with a pharmaceutically acceptable carrier or excipient.

**Patentansprüche für die Vertragsstaaten: BE DE FR GB NL**

1. Verfahren zur Herstellung einer Verbindung der folgenden allgemeinen Formel (I)

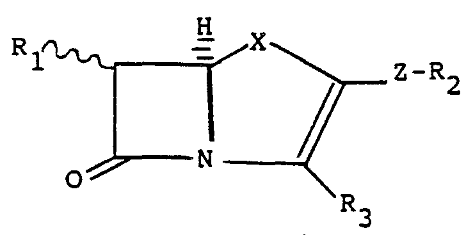

I

in welcher

$R_1$ Wasserstoff, $C_1$—$C_6$-Alkyl, Acylamino oder

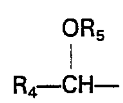

bedeutet, worin $R_4$ Wasserstoff, $C_1$—$C_6$-Alkyl, Aryl, durch $C_1$—$C_6$-Alkyl, $C_1$—$C_6$-Alkoxy oder Halogen

substituiertes Phenyl oder Heteroaryl ist, wobei das Heteroaryl eine Arylgruppe mit einem Heteroatom im Ring und gegebenenfalls 1 bis 3 $C_1$—$C_6$-Alkyl-Substiuenten ist, und $R_5$ für Wasserstoff oder eine Hydroxyschutzgruppe steht;

$R_2$ $C_1$—$C_6$-Alkyl, Aryl, durch $C_1$—$C_6$-Alkyl, $C_1$—$C_6$-Alkoxy oder Halogen substituiertes Phenyl, durch eine oder mehrere Arylgruppen substituiertes $C_1$—$C_6$-Alkyl, Aminoalkyl, N-geschütztes Aminoalkyl, Hydroxyalkyl, O-geschüztes Hydroxyalkyl, einen gegebenenfalls versterten, gegebenenfalls N-geschützten α-Aminosäurerest oder eine gegebenenfalls veresterte Carboxyalkylgruppe bedeutet;

$R_3$ Nitril, Tetrazolyl oder — $COOR_6$ ist, worin $R_6$ Wasserstoff, -Alk-C(Hal)$_3$ (in welcher Hal Halogen ist und Alk ein $C_1$—$C_6$-Alkylenradikal darstellt), $C_1$—$C_6$-Alkyl, Aryl, durch $C_1$—$C_6$-Alkyl, $C_1$—$C_6$-Alkoxy oder Halogen substituiertes Phenyl, eine Allylgruppe, eine *in vivo* metabolisch entfernbare Estergruppe oder eine Carboxyschutzgruppe ist;

X and Z unabhängig voneinander für Schwefel, Sauerstoff, —$CH_2$—, —$CH_2$—$CH_2$ oder den Rest = $NR_7$ stehen, in welchem $R_7$ Wasserstoff, Carbonsäureacyl, $C_1$—$C_6$-Alkyl, Cycloalkyl mit 3 bis 6 Kohlenstoffatomen, Aryl, durch $C_1$—$C_6$-Alkyl, $C_1$—$C_6$-Alkoxy oder Halogen substituiertes Phenyl oder eine N-Schutzgruppe bedeutet; oder eines pharamzeutisch annehmbaren Salzes davon; gekennzeichnet durch Umsetzung einer entsprechenden Verbindung der folgenden allgemeinen Formel (II)

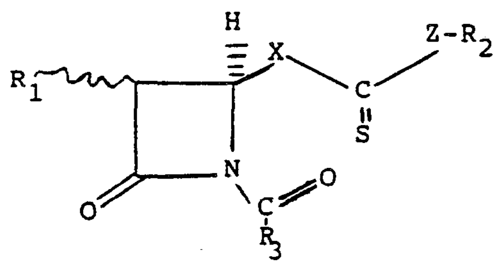

II

in welcher $R_1$, $R_2$, $R_3$, X und Z obige Bedeutung haben, worin $R_5$ eine Hydroxyschutzgruppe ist, $R_7$ nicht Wasserstoff bedeutet, jede Carboxygruppe im Substituenten $R_2$ verestert ist und $R_6$ nicht Wasserstoff bedeutet; mit einem organo-substituierten Derivat von phosphoriger Säure und falls erforderlich oder erwünscht Unterwerfung der entstandenen Verbindung, vor und nach der Abtrennung und etwaiger Auftrennung in ihre sterochemischen Isomeren, falls eine Mischung von Isomeren von Verbindungen der Formel (II) der vorerwähnten Reaktion unterworfen wurde, einem oder mehreren der folgenden Verfahrensschritten:

(a) Entfernung von einer odere mehreren Schutzgruppen;
(b) Umwandlung einer geeigneten Funktion in eine freie Säure;
(c) Umwandlung einer geeigneten Funktion in ein pharmazeutisch annehmbares Salz;
(d) Umwandlung einer geeigneten Funktion in eine metabolisierbare Estergruppe;

mit der Maßgabe, daß wenn $R_1$ Wasserstoff, $C_1$—$C_6$-Alkyl, Hydroxyalkyl, Acyloxyalkyl, Alkylsulfonyloxyalkyl, Arylsulfonyloxyalkyl oder Trialkylsilyloxyalkyl ist, X und Z je für Schwefel stehen, $R_2$ Aminoalkyl oder N-geschütztes Aminoalkyl ist, in welcher $R_2$-Gruppe der Alkylteil verzweigt ist, und $R_3$—$COOR_6$ ist, das organo-substituierte Derivat nicht ein Phosphorigsäuretriester oder ein Phosphorigsäuretriamid sein kann.

2. Verfahren nach Anspruch 1, dadurch gekennzeichnet, daß das organo-substituierte Derivat der phosphorigen Säure ein acyclisches Trialkylphosphit ist.

3. Verfahren nach Anspruch 2, dadurch gekennzeichnet, daß zwei Moläquivalente von Trialkylphosphit pro Mol der Verbindung der Formel (II) verwendet werden, und die Reaktion bei einer Temperatur von 40°C bis 60°C durchgeführt wird.

4. Verfahren nach einem der Ansprüche 1 bis 3, gekennzeichnet durch die Verwendung einer Verbindung der Formel (II), welche durch Reaktion einer Verbindung der folgenden allgemeinen Formel (III)

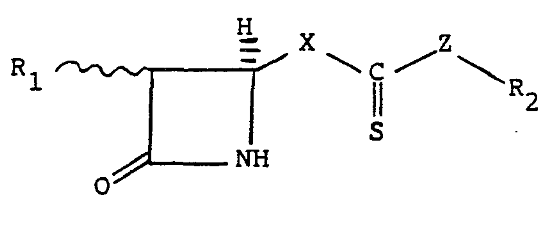

III

in welcher $R_1$, $R_2$, X und Z die in Anspruch 1 im Zusammenhang mit Formel (II) angegebene Bedeutung haben, mit einem reaktiven Derivat einer Säure der folgenden allgemeinen Formel (IV)

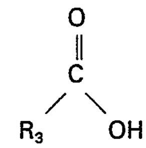

IV

in welcher $R_3$ die in Anspruch 1 angegebene Bedeutung hat, in einem inerten Lösungsmittel in Gegenwart einer organischen Base und, wenn das reaktive Derivat ein Säurehalogenid ist, gegebenenfalls eines Halogenwasserstoffbindemittels hergestellt wurde.

5. Verfahren nach Anspruch 4, gekennzeichnet durch Mischen der Verbindung der Formel (III) und (pro Mol der Verbindung der Formel (III)) ungefähr je eines Moläquivalentes eines Halogenidderivates der Säure der Formel (IV) und eines tertiären Amins, und mindestens eines Moläquivalents eines Halogenwasser stoffbindemittels in dem inerten Lösungsmittel.

6. Verfahren nach Anspruch 4 oder 5, dadurch gekennzeichnet, daß das Säurehalogenid der Formel (IV) Allyloxyoxalylchlorid oder vorzugsweise Chlorallyloxyoxalylchlorid, das tertiäre Amin Diisopropylethylamin und das Bindemittel Kalziumcarbonat ist.

7. Verfahren nach einem der vorhergehenden Ansprüche, dadurch gekennzeichnet, daß eine Verbindung der Formel (I), in welcher X und Z für S stehen, $R_1$

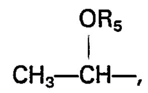

$R_2$ $C_1$—$C_6$-Alkyl und $R_3$ —$COOR_6$ ist, in welcher $R_6$ Allyl oder vorzugsweise Chlorallyl ist, hergestellt wird.

8. Verfahren zur Herstellung einer pharmazeutischen Zusammensetzung, welches umfaßt:

(a) Herstellung einer pharmazeutisch wirksamen Verbindung der Formel (I) gemaß einem der Ansprüche 1 bis 7, und

(b) Mischen der so hergestellten Verbindung mit einem pharmazeutisch annehmbaren Träger oder Bindemittel.

**Patentansprüche für die Vertragsstaaten: AT CH IT LI SE**

1. Verfahren zur Herstellung einer Vebindung der folgenden allgemeinen Formel (I)

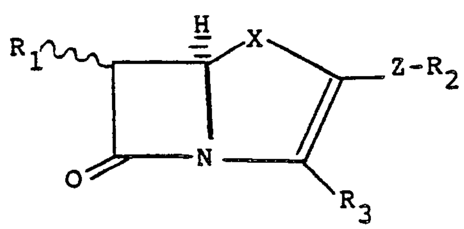

I

in welcher

$R_1$ Wasserstoff, $C_1$—$C_6$-Alkyl, Acylamino oder

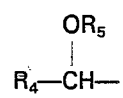

bedeutet, worin $R_4$ Wasserstoff, $C_1$—$C_6$-Alkyl, Aryl, durch $C_1$—$C_6$-Alkyl, $C_1$—$C_6$-Alkoxy oder Halogen substituiertes Phenyl oder Heteroaryl ist, wobei das Heteroaryl eine Arylgruppe mit einem Heteroatom im Ring und gegebenenfalls 1 bis 3 $C_1$—$C_6$-Alkyl-Substituenten ist, und $R_5$ für Wasserstoff oder eine Hydroxy-schutzgruppe steht;

$R_2$ $C_1$—$C_6$-Alkyl, Aryl, durch $C_1$—$C_6$-Alkyl, $C_1$—$C_6$-Alkoxy oder Halogen substituiertes Phenyl, durch eine oder mehrere Arylgruppen substituiertes $C_1$—$C_6$-Alkyl, Aminoalkyl, N-geschütztes Aminoalkyl, Hydroxyalkyl, O-geschütztes Hydroxyalkyl, einen gegebenenfalls veresterten, gegebenenfalls N-geschützten α-Aminosäurerest oder eine gegebenenfalls veresterte Carboxyalkylgruppe bedeutet;

$R_3$ Nitril, Tetrazolyl oder —$COOR_6$ ist, worin $R_6$ Wasserstoff, -Alk-C(Hal)$_3$ (in welcher Hal Halogen ist und Alk ein $C_1$—$C_6$-Alkylenradikal darstellt), $C_1$—$C_6$-Alkyl, Aryl, durch $C_1$—$C_6$-Alkyl, $C_1$—$C_6$-Alkoxy oder Halogen substituiertes Phenyl, eine Allylgruppe, eine *in vivo* metabolisch entfernbare Estergruppe oder eine Carboxyschutzgruppe ist;

X und Z unabhängig voneinander für Schwefel, Sauerstoff, —$CH_2$—, —$CH_2$—$CH_2$ oder den Rest = $NR_7$ stehen in welchem $R_7$ Wasserstoff, Karbonsäureacyl, $C_1$—$C_6$-Alkyl, Cycloalkyl mit 3 bis 6

Kohlenstoffatomen, Aryl, durch $C_1$—$C_6$-Alkyl, $C_1$—$C_6$-Alkoxy oder Halogen substituiertes Phenyl oder eine N-Schutzgruppe bedeutet; oder eines pharmazeutisch annehmbaren Salzes davon; gekennzeichnet durch Umsetzung einer entsprechenden Verbindung der folgenden allgemeinen Formel (II)

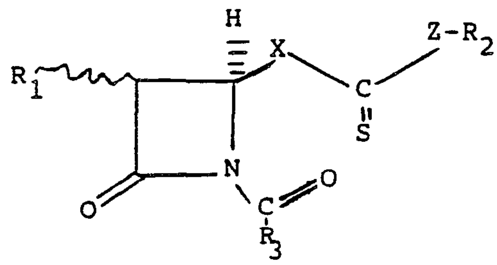

II

in welcher $R_1$, $R_2$, $R_3$, X und Z obige Bedeutung haben, worin $R_5$ eine Hydroxyschutzgruppe ist, $R_7$ nicht Wasserstoff bedeutet, jede Carboxygruppe im Substituenten $R_2$ verestert ist und $R_6$ nicht Wasserstoff bedeutet; mit einem organo-substituierten Derivat von phosphoriger Säure und falls erforderlich oder erwünscht Unterwerfung der entstandenen Verbindung, vor oder nach der Abtrennung und etwaiger Auftrennung in ihre stereochemischen Isomeren, falls eine Mischung von Isomeren von Verbindungen der Formel (II) der vorerwähnten Reaktion unterworfen wurde, einem oder mehreren der folgenden Verfahrensschritten:

(a) Entfernung von einer oder mehreren Schutzgruppen;
(b) Umwandlung einer geeigneten Funktion in eine freie Säure;
(c) Umwandlung einer geeigneten Funktion in ein pharmazeutisch annehmbares Salz; und
(d) Umwandlung einer geeigneten Funktion in eine metabolisierbare Estergruppe;

2. Verfahren nach Anspruch 1, dadurch gekennzeichnet, daß das organo-substituierte Derivat der phosphorigen Säure ein acyclisches Trialkylphosphit ist.

3. Verfahren nach Anspruch 2, dadurch gekennzeichnet, daß zwei Moläquivalente von Trialkylphosphit pro Mol der Verbindung der Formel (II) verwendent werden, und die Reaktion bei einer Temperatur von 40°C bis 60°C durchgeführt wird.

4. Verfahren nach einem der Ansprüche 1 bis 3, gekennzeichnet durch die Verwendung einer Verbindung der Formel (II), welche durch Reaktion einer Verbindung der folgenden allgemeinen Formel (III)

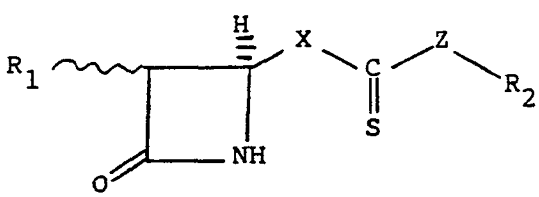

III

in welcher $R_1$, $R_2$, X und Z die in Anspruch 1 im Zusammenhang mit Formel (II) angegebene Bedeutung haben, mit einem reaktiven Derivat einer Säure der folgenden allgemeinen Formel (IV)

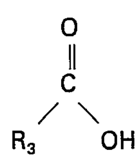

IV

in welcher $R_3$ die in Anspruch 1 angegebene Bedeutung hat, in einem inerten Lösungsmittel in Gegenwart einer organischen Base und, wenn das reaktive Derivat ein Säurehalogenid ist, gegebenenfalls eines Halogenwasserstoffbindemittels hergestellt wurde.

5. Verfahren nach Anspruch 4, gekennzeichnet durch Mischen der Verbindung der Formel (III) und (pro Mol der Verbindung der Formel (III)) ungefähr je eines Moläquivalentes eines Halogenidderivates der Säure der Formel (IV) und eines tertiären Amins, und mindestens eines Moläquivalents eines Halogenwasser stoffbindemittels in dem inerten Lösungsmittel.

6. Verfahren nach Anspruch 4 oder 5, dadurch gekennzeichnet, daß das Säurehalogenid der Formel (IV) Allyloxyoxalylchlorid oder vorzugsweise Chlorallyloxyoxalylchlorid, das tertiäre Amin Diisopropylethylamin und das Bindemittel Kalziumcarbonat ist.

7. Verfahren nach einem der vorhergehenden Ansprüche, dadurch gekennzeichnet, daß eine Verbindung der Formel (I), in welcher X und Z für S stehen, $R_1$

$$\begin{array}{c} OR_5 \\ | \\ CH_3—CH—, \end{array}$$

$R_2$ $C_1$—$C_6$-Alkyl und $R_3$ —$COOR_6$ ist, in welcher $R_6$ Allyl oder vorzugsweise Chlorallyl ist, hergestellt wird.

8. Verfahren zur Herstellung einer pharmazeutischen Zusammensetzung, welches umfaßt:

(a) Herstellung einer pharmazeutisch wirksamen Verbindung der Formel (I) gemaß einem der Ansprüche 1 bis 7, und

(b) Mischen der so hergestelten Verbindung mit einem pharmazeutisch annehmbaren Träger oder Bindemittel.

**Revendications pour les Etats Contractants: BE DE FR GB NL**

1. Procédè de préparation d'un composé de formule générale (I) suivante

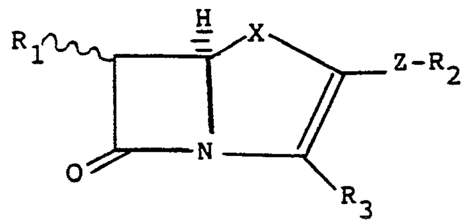

I

dans laquelle

$R_1$ est hydrogène, alcoyle $C_1$—$C_6$, acylamino ou

$$\begin{array}{c} OR_5 \\ | \\ R_4—CH—, \end{array}$$

où $R_4$ est hydrogène, alcoyle $C_1$—$C_6$, aryle, phényle substitué par un alcoyle $C_1$—$C_6$, par un alcoxy $C_1$—$C_6$ ou par un halogène ou hétéroaryle, ledit hétéroaryle étant un groupe aryle ayant un hétéroatome dans le noyau et ayant éventuellement 1 à 3 substituants alcoyles $C_1$—$C_6$, et $R_5$ est hydrogène ou un groupe hydroxy protecteur;

$R_2$ est alcoyle $C_1$—$C_6$, aryle, phényle substitué par un alcoyle $C_1$—$C_6$, par un alcoxy $C_1$—$C_6$ ou par un halogène, alcoyle $C_1$—$C_6$ substitué par un ou plusieurs groupes aryles, aminoalcoyle, aminoalcoyle *N*-protégé, hydroxyalcoyle, hydroxyalcoyle O-protégé, un résidu d'α-amino-acide éventuellement estérifié, éventuellement N-protégé ou un groupe carboxyalcoyle éventuellement estérifié;

$R_3$ est nitrile, tétrazolyle ou —$COOR_6$, où $R_6$ est hydrogène, -Alk-C(Hal)$_3$ (où Hal est halogène et Alk représente un radical alcoylène $C_1$—$C_6$), alcoyle $C_1$—$C_6$, aryle, phényle substitué par un alcoyle $C_1$—$C_6$, par un alcoxy $C_1$—$C_6$ ou par un halogène, un groupe allyle, un groupe ester qui peut être métaboliquement retiré *in vivo,* ou un groupe carboxy protecteur;

X et Z sont indépendamment du soufre, de l'oxygène, —$CH_2$—, —$CH_2$—$CH_2$ ou le radical =$NR_7$ où $R_7$ est hydrogène, acyle d'acide carboxylique, alcoyle $C_1$—$C_6$, cycloalcoyle contenant 3 à 6 atomes de carbone, aryle, phényle substitué par un alcoyle $C_1$—$C_6$, par un alcoxy $C_1$—$C_6$ ou par un halogène ou un groupe *N*-protecteur;

ou son sel acceptable en pharmacie;

caractérisé par la réaction d'un composé approprié de formule génerale (II)

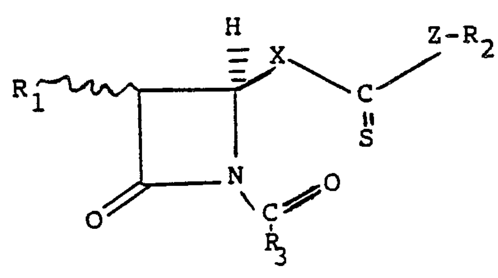

II

où $R_1$, $R_2$, $R_3$, X et Z sont tels que définis ci-dessus, où $R_5$ est un groupe hydroxy protecteur, $R_7$ n'est pas de l'hydrogène, tout groupe carboxy dans le substituant $R_2$ est estérifié et $R_6$ n'est pas de l'hydrogène; avec

un dérivé organo-substitué de l'acide phosphoreux, et si requis ou si on le souhaite, en soumettant le composé résultant, avant de ou subséquemment à l'isolement et toute séparation en ses isomères stéréochimiques, si un mélange d'isomères des composés de formule (II) a été soumis à la réaction ci-dessus, à une ou plusieurs des opérations suivantes:

(a) enlèvement d'un ou plusieurs groupes protecteurs;

(b) conversion d'une fonction appropriée en un acide libre;

(c) conversion d'une fonction appropriée en un sel acceptable en pharmacie; et

(d) conversion d'une fonction appropriée en un groupe ester métabolisable;

à condition que quand $R_1$ est hydrogène, alcoyle $C_1$—$C_6$, hydroxyalcoyle, acyloxyalcoyle, alkylsulfonyloxyalcoyle, aryl sulfonyloxyalcoyle ou trialkylsilyloxyalcoyle, X et Z sont tous deux du soufre, $R_2$ est aminoalcoyle ou aminoalcoyle N-protégé, groupe $R_2$ dans lequel le fragment alcoyle est ramifié, et $R_3$ est —$COOR_6$, alors le dérivé organo-substitué ne puisse être un triester de l'acide phosphoreux ou un triamide de l'acide phosphoreux.

2. Méthode selon la revendication 1 caractérisée en ce que le dérivé organo-substitué de l'acide phosphoreux est un trialkylphosphite acyclique.

3. Procédé selon la revendication 2 caractérisé en ce que deux équivalents molaires de trialkylphosphite sont utilisés par mole du composé de formule (II), et la réaction est accomplie à une température de 40°C à 60°C.

4. Procédé selon l'une quelconque des revendications 1 à 3 caractérisé en ce qu'on utilise un composé de formule (II) qui a été préparé par la réaction d'un composé ayant la formule générale (III) qui suit

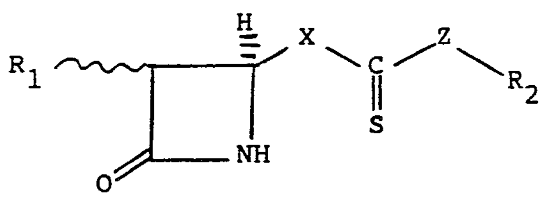

III

dans laquelle $R_1$, $R_2$, X et Z sont tels que définis à la revendication 1 par rapport à la formule (II), avec un dérivé réactif d'un acide de la formule générale (IV) qui suit

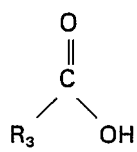

IV

dans laquelle $R_3$ est tel que défini à la revendication 1, dans un solvant inerte, en présence d'une base organique et, lorsque le dérivé réactif est un halogénure d'acide, éventuellement un agent liant l'halogénure d'hydrogène.

5. Procédé selon la revendication 4 caractérisé en ce qu'on mélange, dans ledit solvant inerte, le composé de formule (III) et (par mole du composé de formule (III)), environ un équivalent molaire de chacun d'un dérivé d'halogénure de l'acide de formule (IV) et d'une amine tertiaire, et au moins un équivalent molaire de l'agent liant l'halogénure d'hydrogène.

6. Procédé selon la revendication 4 ou 5 caractérisé en ce que l'halogénure d'acide de la formule (IV) est le chlorure d'allyloxyoxalyle ou de préférence l'oxyallylchlorure de chloroallyloxy, l'amine tertiaire est la di-isopropyléthylamine et l'agent liant est le carbonate de calcium.

7. Procédé selon l'une quelconque des revendications précédentes caractérisé en ce qu'un composé de formule (I), où X et Z sont tous deux S, $R_1$ est

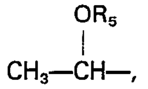

$R_2$ est alcoyle $C_1$—$C_6$ et $R_3$ est —$COOR_6$ où $R_6$ est allyle ou de préférence chloroallyle, est produit.

8. Méthode de préparation d'une composition pharmaceutique qui comprend:

a) la préparation d'un composé pharmaceutiquement actif de formule (I) selon l'une quelconque des revendications 1 à 7, et

b) le mélange du composé ainsi préparé avec un véhicule ou excipient acceptable en pharmacie.

**Revendications pour les Etats contractants: AT CH IT LI SE**

1. Procédé de préparation d'un composé de formule générale (I) suivante

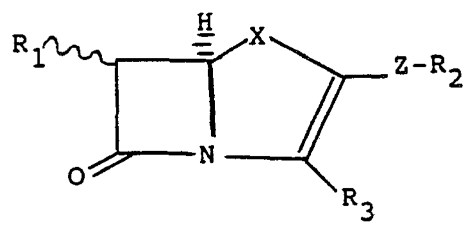

I

dans laquelle

$R_1$ est hydrogène, alcoyle $C_1$—$C_6$, acylamino ou

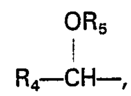

où $R_4$ est hydrogène, alcoyle $C_1$—$C_6$, aryle, phényle substitué par un alcoyle $C_1$—$C_6$, par un alcoxy $C_1$—$C_6$ ou par un halogène ou hétéroaryle, ledit hétéroaryle étant un groupe aryle ayant un hétéroatome dans le noyau et ayant éventuellement 1 à 3 substituants alcoyles $C_1$—$C_6$, et $R_5$ est hydrogène ou un groupe hydroxy protecteur;

$R_2$ est alcoyle $C_1$—$C_6$, aryle, phényle substitué par un alcoyle $C_1$—$C_6$, par un alcoxy $C_1$—$C_6$ ou par un halogène, alcoyle $C_1$—$C_6$ substitué par un ou plusieurs groupes aryles, aminoalcoyle, aminoalcoyle *N*-protégé, hydroxyalcoyle, hydroxyalcoyle O-protégé, un résidu d'α-amino-acide éventuellement estérifié, éventuellement N-protégé ou un groupe carboxyalcoyle éventuellement estérifié;

$R_3$ est nitrile, tétrazolyle ou —$COOR_6$, où $R_6$ est hydrogène, -Alk-C(Hal)$_3$ (où Hal est halogène et Alk représente un radical alcoylène $C_1$—$C_6$), alcoyle $C_1$—$C_6$, aryle, phényle substitué par un alcoyle $C_1$—$C_6$, par un alcoxy $C_1$—$C_6$ ou par un halogène, un groupe allyle, un groupe ester qui peut être métaboliquement retiré *in vivo,* ou un groupe carboxy protecteur;

X et Z sont indépendamment du soufre, de l'oxygène, —$CH_2$—, —$CH_2$—$CH_2$ ou le radical $=NR_7$ où $R_7$ est hydrogène, acyle d'acide carboxylique, alcoyle $C_1$—$C_6$, cycloalcoyle contenant 3 à 6 atomes de carbone, aryle, phényle substitué par un alcoyle $C_1$—$C_6$, par un alcoxy $C_1$—$C_6$ ou par un halogène ou un groupe *N*-protecteur;

ou son sel acceptable en pharmacie;

caractérisé par la réaction d'un composé approprié de formule générale (II)

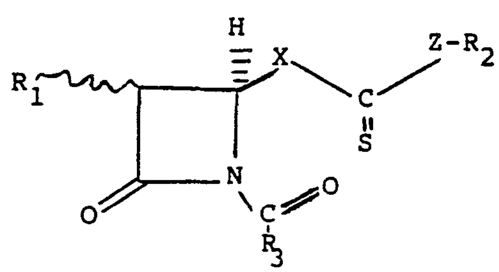

II

où $R_1$, $R_2$, $R_3$, X et Z sont tels que définis ci-dessus, où $R_5$ est un groupe hydroxy protecteur, $R_7$ n'est pas de l'hydrogène, tout groupe carboxy dans le substituant $R_2$ est estérifié et $R_6$ n'est pas de l'hydrogène; avec un dérivé organo-substitué de l'acide phosphoreux, et si requis ou si on le souhaite, en soumettant le composé résultant, avant de ou subséquemment à l'isolement et toute séparation en ses isomères stéréochimiques, si un mélange d'isomères des composés de formule (II) a été soumis à la réaction ci-dessus, à une ou plusieurs des opérations suivantes:

(a) enlèvement d'un ou plusieurs groupes protecteurs;

(b) conversion d'une fonction appropriée en un acide libre;

(c) conversion d'une fonction appropriée en un sel acceptable en pharmacie; et

(d) conversion d'une fonction appropriée en un groupe ester métabolisable;

2. Méthode selon la revendication 1 caractérisée en ce que le dérivé organo-substitué de l'acide phosphoreux est un trialkylphosphite acyclique.

3. Procédé selon la revendication 2 caractérisé en ce que deux équivalents molaires de trialkylphosphite sont utilisés par mole du composé de formule (II), et la réaction est accomplie à une température de 40°C à 60°C.

4. Procédé selon l'une quelconque des revendications 1 à 3 caractérisé en ce qu'on utilise un composé de formule (II) qui a été préparé par la réaction d'un composé ayant la formule générale (III) qui suit

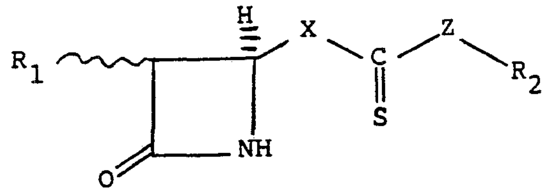

III

dans laquelle $R_1$, $R_2$, X et Z sont tels que définis à la revendication 1 par rapport à la formule (II), avec un dérivé réactif d' un acide de la formule générale (IV) qui suit

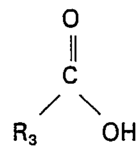

IV

dans laquelle $R_3$ est tel que défini à la revendication 1, dans un solvant inerte, en présence d'une base organique et, lorsque le dérivé réactif est un halogénure d'acide, éventuellement un agent liant l'halogénure d'hydrogène.

5. Procédé selon la revendication 4 caractérisé en ce qu'on mélange, dans ledit solvant inerte, le composé de formule (III) et (par mole du composé de formule (III)), environ un équivalent molaire de chacun d'un dérivé d'halogénure de l'acide de formule (IV) et d'une amine tertiaire, et au moins un équivalent molaire de l'agent liant l'halogénure d'hydrogène.

6. Procédé selon la revendication 4 ou 5 caractérisé en ce que l'halogénure d'acide de la formule (IV) est le chlorure d'allyloxyoxalyle ou de préférence l'oxyallylchlorure de chloroallyloxy, l'amine tertiaire est la di-isopropyléthylamine et l'agent liant est le carbonate de calcium.

7. Procédé selon l'une quelconque des revendications précédentes caractérisé en ce qu'un composé de formule (I), où X et Z sont tous deux S, $R_1$ est

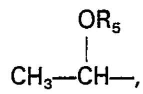

$R_2$ est alcoyle $C_1$—$C_6$ et $R_3$ est —$COOR_6$ où $R_6$ est allyle ou de préférence chloroallyle, est produit.

8. Méthode de préparation d'une composition pharmaceutique qui comprend:

a) la préparation d'un composé pharmaceutiquement actif de formule (I) selon l'une quelconque des revendications 1 à 7, et

b) le mélange du composé ainsi préparé avec un véhicule ou excipient acceptable en pharmacie.